# EUROPEAN PATENT SPECIFICATION

(11) **EP 4 213 718 B1**
(45) Date of publication and mention of the grant of the patent: **20.08.2025**
(21) Application number: 21786578.1
(22) Date of filing: 17.09.2021
(51) Int. Cl.: A61B 5/08, A61B 5/16, A61B 5/00, A61M 16/00

(54) **SYSTEMS AND METHODS FOR AIDING A RESPIRATORY THERAPY SYSTEM USER**
SYSTEME UND VERFAHREN ZUR UNTERSTÜTZUNG EINES ATEMTHERAPIESYSTEMBENUTZERS
SYSTÈMES ET PROCÉDÉS POUR AIDER UN UTILISATEUR D'UN SYSTÈME DE THÉRAPIE RESPIRATOIRE

(30) Priority: 18.09.2020 US 202063080401 P
(43) Date of publication of application: 26.07.2023
(73) Proprietor: ResMed Sensor Technologies Limited, Sandyford D18 T6T7 Dublin (IE)
(72) Inventor: LYON, Graeme Alexander, Dublin, D18 T6T7 (IE); SHOULDICE, Redmond, Dublin, D18 T6T7 (IE); RICE, Anna, Dublin, D18 T6T7 (IE); FOX, Niall Andrew, Dublin, D18 T6T7 (IE)
(74) Representative: Mathys & Squire
(86) International application number: PCT/IB2021/058518
(87) International publication number: WO 2022/058967

(56) References cited:
- CA-A1- 2 623 661
- US-A1- 2005 061 319
- US-A1- 2009 260 630
- US-A1- 2017 239 433

## Description

### CROSS-REFERENCE TO RELATED APPLICATIONS

This application claims the benefit of and priority to U.S. Provisional Patent Application No. 63/080,401, filed September 18, 2020.

### TECHNICAL FIELD

The present disclosure relates generally to systems and methods for aiding a user in using a respiratory therapy system, and more particularly, to systems and methods for determining an emotion score associated with a user and communicating one or more prompts to the user to aid in modifying the emotion score.

### BACKGROUND

Many individuals suffer from sleep-related and/or respiratory disorders such as, for example, Periodic Limb Movement Disorder (PLMD), Restless Leg Syndrome (RLS), Sleep-Disordered Breathing (SDB) such as Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), chest wall disorders, and insomnia. Many of these disorders can be treated using a respiratory therapy system, while others may be treated using a different technique. However, the user may experience anxiety or stress when using the respiratory therapy system, for example, due to unfamiliarity (e.g., a first-time user) or discomfort. This anxiety or stress can inhibit the user from falling asleep or cause the user to abandon using the respiratory therapy system. The present disclosure is directed to solving these and other problems. CA2623661A1 discloses a respiratory therapy system with detection for an emotional state.

### SUMMARY

According to some implementations of the present disclosure, a method includes receiving first physiological data associated with a user. The method also includes determining a first emotion score associated with the user based at least in part on the first physiological data. The method also includes, responsive to determining that the first emotion score satisfies a predetermined condition, modifying one or more settings of a respiratory therapy system.

According to some implementations of the present disclosure, a system includes an electronic interface, a memory, and a control system. The electronic interface is configured to receive physiological data associated with a user. The memory stores machine-readable instructions. The control system includes one or more processors configured to execute the machine-readable instructions to determine an emotion score associated with the user based at least in part on the physiological data. The control system is further configured to modify one or more settings of a respiratory therapy system responsive to determining that the emotion score satisfies a predetermined condition.

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system, according to some implementations of the present disclosure;
FIG. 2 is a perspective view of at least a portion of the system of FIG. 1, a user, and a bed partner, according to some implementations of the present disclosure;
FIG. 3A is a perspective view of a user interface of the respiratory therapy system of FIG. 1, according to some implementations of the present disclosure;
FIG. 3B is a perspective exploded view of the user interface of FIG. 3A, according to some implementations of the present disclosure;
FIG. 4 illustrates an exemplary timeline for a sleep session, according to some implementations of the present disclosure;
FIG. 5 illustrates an exemplary hypnogram associated with the sleep session of FIG. 4, according to some implementations of the present disclosure; and
FIG. 6 is a process flow diagram for a method for aiding a user, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the scope of the present disclosure. The invention is defined solely by the appended claims.

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB) such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as Central Sleep Apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd., the disclosure of each of which is hereby incorporated by reference herein in their entireties.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that can occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

Additionally, many individuals suffer from insomnia, a condition which is generally characterized by a dissatisfaction with sleep quality or duration (e.g., difficulty initiating sleep, frequent or prolonged awakenings after initially falling asleep, and an early awakening with an inability to return to sleep). It is estimated that over 2.6 billion people worldwide experience some form of insomnia, and over 750 million people worldwide suffer from a diagnosed insomnia disorder. In the United States, insomnia causes an estimated gross economic burden of $107.5 billion per year, and accounts for 13.6% of all days out of role and 4.6% of injuries requiring medical attention. Recent research also shows that insomnia is the second most prevalent mental disorder, and that insomnia is a primary risk factor for depression.

Nocturnal insomnia symptoms generally include, for example, reduced sleep quality, reduced sleep duration, sleep-onset insomnia, sleep-maintenance insomnia, late insomnia, mixed insomnia, and/or paradoxical insomnia. Sleep-onset insomnia is characterized by difficulty initiating sleep at bedtime. Sleep-maintenance insomnia is characterized by frequent and/or prolonged awakenings during the night after initially falling asleep. Late insomnia is characterized by an early morning awakening (e.g., prior to a target or desired wakeup time) with the inability to go back to sleep. Comorbid insomnia refers to a type of insomnia where the insomnia symptoms are caused at least in part by a symptom or complication of another physical or mental condition (e.g., anxiety, depression, medical conditions, and/or medication usage). Mixed insomnia refers to a combination of attributes of other types of insomnia (e.g., a combination of sleep-onset, sleep-maintenance, and late insomnia symptoms). Paradoxical insomnia refers to a disconnect or disparity between the user's perceived sleep quality and the user's actual sleep quality.

Diurnal (e.g., daytime) insomnia symptoms include, for example, fatigue, reduced energy, impaired cognition (e.g., attention, concentration, and/or memory), difficulty functioning in academic or occupational settings, and/or mood disturbances. These symptoms can lead to psychological complications such as, for example, lower mental (and/or physical) performance, decreased reaction time, increased risk of depression, and/or increased risk of anxiety disorders. Insomnia symptoms can also lead to physiological complications such as, for example, poor immune system function, high blood pressure, increased risk of heart disease, increased risk of diabetes, weight gain, and/or obesity.

Co-morbid Insomnia and Sleep Apnea (COMISA) refers to a type of insomnia where the subject experiences both insomnia and obstructive sleep apnea (OSA). OSA can be measured based on an Apnea-Hypopnea Index (AHI) and/or oxygen desaturation levels. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild OSA. An AHI that is greater than or equal to 15, but less than 30 is considered indicative of moderate OSA. An AHI that is greater than or equal to 30 is considered indicative of severe OSA. In children, an AHI that is greater than 1 is considered abnormal.

Insomnia can also be categorized based on its duration. For example, insomnia symptoms are considered acute or transient if they occur for less than 3 months. Conversely, insomnia symptoms are considered chronic or persistent if they occur for 3 months or more, for example. Persistent/chronic insomnia symptoms often require a different treatment path than acute/transient insomnia symptoms.

Known risk factors for insomnia include gender (e.g., insomnia is more common in females than males), family history, and stress exposure (e.g., severe and chronic life events). Age is a potential risk factor for insomnia. For example, sleep-onset insomnia is more common in young adults, while sleep-maintenance insomnia is more common in middle-aged and older adults. Other potential risk factors for insomnia include race, geography (e.g., living in geographic areas with longer winters), altitude, and/or other sociodemographic factors (e.g. socioeconomic status, employment, educational attainment, self-rated health, etc.).

Mechanisms of insomnia include predisposing factors, precipitating factors, and perpetuating factors. Predisposing factors include hyperarousal, which is characterized by increased physiological arousal during sleep and wakefulness. Measures of hyperarousal include, for example, increased levels of cortisol, increased activity of the autonomic nervous system (e.g., as indicated by increase resting heart rate and/or altered heart rate), increased brain activity (e.g., increased EEG frequencies during sleep and/or increased number of arousals during REM sleep), increased metabolic rate, increased body temperature and/or increased activity in the pituitary-adrenal axis. Precipitating factors include stressful life events (e.g., related to employment or education, relationships, etc.) Perpetuating factors include excessive worrying about sleep loss and the resulting consequences, which may maintain insomnia symptoms even after the precipitating factor has been removed.

Conventionally, diagnosing or screening insomnia (including identifying a type or insomnia and/or specific symptoms) involves a series of steps. Often, the screening process begins with a subjective complaint from a patient (e.g., they cannot fall or stay sleep).

Next, the clinician evaluates the subjective complaint using a checklist including insomnia symptoms, factors that influence insomnia symptoms, health factors, and social factors. Insomnia symptoms can include, for example, age of onset, precipitating event(s), onset time, current symptoms (e.g., sleep-onset, sleep-maintenance, late insomnia), frequency of symptoms (e.g., every night, episodic, specific nights, situation specific, or seasonal variation), course since onset of symptoms (e.g., change in severity and/or relative emergence of symptoms), and/or perceived daytime consequences. Factors that influence insomnia symptoms include, for example, past and current treatments (including their efficacy), factors that improve or ameliorate symptoms, factors that exacerbate insomnia (e.g., stress or schedule changes), factors that maintain insomnia including behavioral factors (e.g., going to bed too early, getting extra sleep on weekends, drinking alcohol, etc.) and cognitive factors (e.g., unhelpful beliefs about sleep, worry about consequences of insomnia, fear of poor sleep, etc.). Health factors include medical disorders and symptoms, conditions that interfere with sleep (e.g., pain, discomfort, treatments), and pharmacological considerations (e.g., alerting and sedating effects of medications). Social factors include work schedules that are incompatible with sleep, arriving home late without time to wind down, family and social responsibilities at night (e.g., taking care of children or elderly), stressful life events (e.g., past stressful events may be precipitants and current stressful events may be perpetuators), and/or sleeping with pets.

After the clinician completes the checklist and evaluates the insomnia symptoms, factors that influence the symptoms, health factors, and/or social factors, the patient is often directed to create a daily sleep diary and/or fill out a questionnaire (e.g., Insomnia Severity Index or Pittsburgh Sleep Quality Index). Thus, this conventional approach to insomnia screening and diagnosis is susceptible to error(s) because it relies on subjective complaints rather than objective sleep assessment. There may be a disconnect between patient's subjective complaint(s) and the actual sleep due to sleep state misperception (paradoxical insomnia).

In addition, the conventional approach to insomnia diagnosis does not rule out other sleep-related disorders such as, for example, Periodic Limb Movement Disorder (PLMD), Restless Leg Syndrome (RLS), Sleep-Disordered Breathing (SDB), Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders. These other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping. While these other sleep-related disorders may have similar symptoms as insomnia, distinguishing these other sleep-related disorders from insomnia is useful for tailoring an effective treatment plan distinguishing characteristics that may call for different treatments. For example, fatigue is generally a feature of insomnia, whereas excessive daytime sleepiness is a characteristic feature of other disorders (e.g., PLMD) and reflects a physiological propensity to fall asleep unintentionally. Insomnia can also be tied to emotion (e.g., anxiety-induced insomnia), thus tracking emotion (or anxiety) of the patient can provide insight to managing or treating insomnia.

Once diagnosed, insomnia can be managed or treated using a variety of techniques or providing recommendations to the patient. Generally, the patient can be encouraged or recommended to generally practice healthy sleep habits (e.g., plenty of exercise and daytime activity, have a routine, no bed during the day, eat dinner early, relax before bedtime, avoid caffeine in the afternoon, avoid alcohol, make bedroom comfortable, remove bedroom distractions, get out of bed if not sleepy, try to wake up at the same time each day regardless of bed time) or discouraged from certain habits (e.g., do not work in bed, do not go to bed too early, do not go to bed if not tired). The patient can additionally or alternatively be treated using sleep medicine and medical therapy such as prescription sleep aids, over-the-counter sleep aids, and/or at-home herbal remedies.

The patient can also be treated using cognitive behavior therapy (CBT) or cognitive behavior therapy for insomnia (CBT-I), which generally includes sleep hygiene education, relaxation therapy, stimulus control, sleep restriction, and sleep management tools and devices. Sleep restriction is a method designed to limit time in bed (the sleep window or duration) to actual sleep, strengthening the homeostatic sleep drive. The sleep window can be gradually increased over a period of days or weeks until the patient achieves an optimal sleep duration. Stimulus control includes providing the patient a set of instructions designed to reinforce the association between the bed and bedroom with sleep and to reestablish a consistent sleep-wake schedule (e.g., go to bed only when sleepy, get out of bed when unable to sleep, use the bed for sleep only (e.g., no reading or watching TV), wake up at the same time each morning, no napping, etc.) Relaxation training includes clinical procedures aimed at reducing autonomic arousal, muscle tension, and intrusive thoughts that interfere with sleep (e.g., using progressive muscle relaxation). Cognitive therapy is a psychological approach designed to reduce excessive worrying about sleep and reframe unhelpful beliefs about insomnia and its daytime consequences (e.g., using Socratic question, behavioral experiences, and paradoxical intention techniques). Sleep hygiene generally refers to the individual's practices (e.g., die, exercise, substance use, bedtime, activities before going to sleep, activities in bed before going to sleep, etc.) and/or environmental parameters (e.g., ambient light, ambient noise, ambient temperature, etc.). In at least some cases, the individual can improve their sleep hygiene by going to bed at a certain bedtime each night, sleeping for a certain duration, waking up at a certain time, modifying the environmental parameters, or any combination thereof. Sleep hygiene education includes general guidelines about health practices (e.g., diet, exercise, substance use) and environmental factors (e.g., light, noise, excessive temperature) that may interfere with sleep. Mindfulness-based interventions can include, for example, meditation.

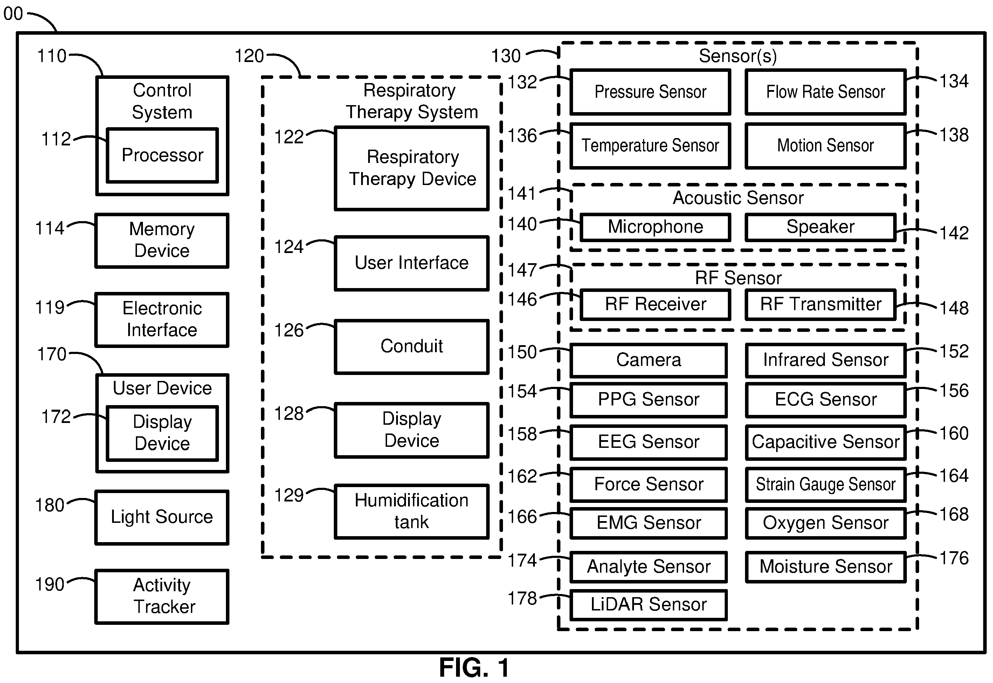

Referring to FIG. 1, a system 100, according to some implementations of the present disclosure, is illustrated. The system 100 includes a control system 110, a memory device 114, an electronic interface 119, a respiratory therapy system 120, one or more sensors 130, one or more user devices 170, a light source 180, and an activity tracker 190.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, a portion (e.g., a housing) of the respiratory therapy system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122, within a housing of the user device 170, the activity tracker 190, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a geographic location of the user, a relationship status, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The medical information data can include results from one or more of a polysomnography (PSG) test, a CPAP titration, or a home sleep test (HST), respiratory therapy system settings from one or more sleep sessions, sleep related respiratory events from one or more sleep sessions, or any combination thereof. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The received data, such as physiological data, flow rate data, pressure data, motion data, acoustic data, etc., may be used to determine and/or calculate physiological parameters. The electronic interface 119 can communicate with the one or more sensors 130 using a wired connection or a wireless connection (e.g., using an RF communication protocol, a Wi-Fi communication protocol, a Bluetooth communication protocol, an IR communication protocol, over a cellular network, over any other optical communication protocol, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 optionally includes a respiratory therapy system 120. The respiratory therapy system 120 can include a respiratory pressure therapy (RPT) device 122 (referred to herein as respiratory therapy device 122), a user interface 124, a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory therapy device 122 is generally used to generate pressurized air that is delivered to a user (e.g., using one or more motors that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver pressurized air at a pressure of at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Generally, the user interface 124 engages the user's face such that the pressurized air is delivered to the user's airway via the user's mouth, the user's nose, or both the user's mouth and nose. Together, the respiratory therapy device 122, the user interface 124, and the conduit 126 form an air pathway fluidly coupled with an airway of the user. The pressurized air also increases the user's oxygen intake during sleep.

Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cm H₂O.

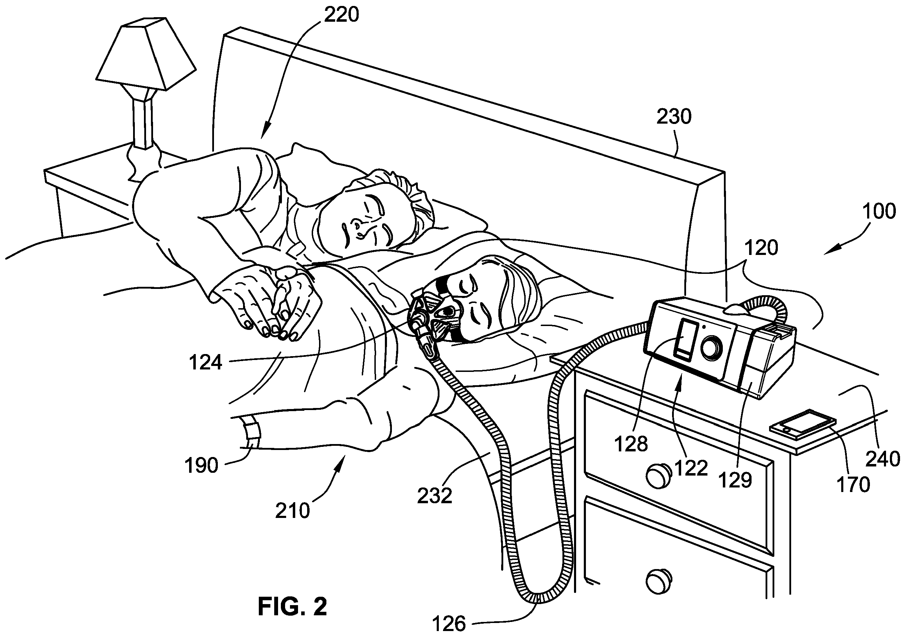

As shown in FIG. 2, in some implementations, the user interface 124 is or includes a facial mask (e.g., a full face mask) that covers the nose and mouth of the user. Alternatively, in some implementations, the user interface 124 is a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. In some examples, the user interface 124 can be a tube-up mask, wherein straps of the mask are configured to act as conduit(s) to deliver pressurized air to the face or nasal mask. The user interface 124 can also include one or more vents for permitting the escape of carbon dioxide and other gases exhaled by the user 210. In other implementations, the user interface 124 includes a mouthpiece (e.g., a night guard mouthpiece molded to conform to the user's teeth, a mandibular repositioning device, etc.).

The conduit 126 (also referred to as an air circuit or tube) allows the flow of air between two components of the respiratory therapy system 120, such as the respiratory therapy device 122 and the user interface 124. In some implementations, there can be separate limbs of the conduit for inhalation and exhalation. In other implementations, a single limb conduit is used for both inhalation and exhalation.

One or more of the respiratory therapy device 122, the user interface 124, the conduit 126, the display device 128, and the humidification tank 129 can contain one or more sensors (e.g., a pressure sensor, a flow rate sensor, a humidity sensor, a temperature sensor, or more generally any of the other sensors 130 described herein). These one or more sensors can be used, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score and/or a therapy score (such as a myAir^{™} score, such as described in WO 2016/061629, which is hereby incorporated by reference herein in its entirety), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122. The humidification tank 129 includes a reservoir of water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself. In other implementations, the respiratory therapy device 122 or the conduit 126 can include a waterless humidifier. The waterless humidifier can incorporate sensors that interface with other sensor positioned elsewhere in system 100.

The respiratory therapy system 120 can be used, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), high-flow therapy (HFT) system, or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure. The HFT system typically provides a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 is a facial mask (e.g., a full face mask) that covers the nose and mouth of the user 210. Alternatively, the user interface 124 can be a nasal mask that provides air to the nose of the user 210 or a nasal pillow mask that delivers air directly to the nostrils of the user 210. The user interface 124 can include a plurality of straps (e.g., including hook and loop fasteners) for positioning and/or stabilizing the interface on a portion of the user 210 (e.g., the face) and a conformal cushion (e.g., silicone, plastic, foam, etc.) that aids in providing an air-tight seal between the user interface 124 and the user 210. The user interface 124 can also include one or more vents for permitting the escape of carbon dioxide and other gases exhaled by the user 210. In other implementations, the user interface 124 is a mouthpiece (e.g., a night guard mouthpiece molded to conform to the user's teeth, a mandibular repositioning device, etc.) for directing pressurized air into the mouth of the user 210.

The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can be positioned on a nightstand 240 that is directly adjacent to the bed 230 as shown in FIG. 2, or more generally, on any surface or structure that is generally adjacent to the bed 230 and/or the user 210.

Generally, a user who is prescribed usage of the respiratory therapy system 120 will tend to experience higher quality sleep and less fatigue during the day after using the respiratory therapy system 120 during the sleep compared to not using the respiratory therapy system 120 (especially when the user suffers from sleep apnea or other sleep related disorders). For example, the user 210 may suffer from obstructive sleep apnea and rely on the user interface 124 (e.g., a full face mask) to deliver pressurized air from the respiratory therapy device 122 via conduit 126. The respiratory therapy device 122 can be a continuous positive airway pressure (CPAP) machine used to increase air pressure in the throat of the user 210 to prevent the airway from closing and/or narrowing during sleep. For someone with sleep apnea, their airway can narrow or collapse during sleep, reducing oxygen intake, and forcing them to wake up and/or otherwise disrupt their sleep. The CPAP machine prevents the airway from narrowing or collapsing, thus minimizing the occurrences where she wakes up or is otherwise disturbed due to reduction in oxygen intake. While the respiratory therapy device 122 strives to maintain a medically prescribed air pressure or pressures during sleep, the user can experience sleep discomfort due to the therapy.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a Light Detection and Ranging (LiDAR) sensor 178, an electrodermal sensor, an accelerometer, an electrooculography (EOG) sensor, a light sensor, a humidity sensor, an air quality sensor, or any combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

As described herein, the system 100 generally can be used to generate data (e.g., physiological data, flow rate data, pressure data, motion data, acoustic data, etc.) associated with a user (e.g., a user of the respiratory therapy system 120 shown in FIG. 2) before, during, and/or after a sleep session. The generated data can be analyzed to generate one or more physiological parameters (e.g., before, during, and/or after a sleep session) and/or sleep-related parameters (e.g., during a sleep session), which can include any parameter, measurement, etc. related to the user. Examples of the one or more physiological parameters include a respiration pattern, a respiration rate, an inspiration amplitude, an expiration amplitude, a heart rate, heart rate variability, a length of time between breaths, a time of maximal inspiration, a time of maximal expiration, a forced breath parameter (e.g., distinguishing releasing breath from forced exhalation), respiration variability, breath morphology (e.g., the shape of one or more breaths), movement of the user 210, temperature, EEG activity, EMG activity, ECG data, a sympathetic response parameter, a parasympathetic response parameter, and the like. The one or more sleep-related parameters that can be determined for the user 210 during the sleep session include, for example, an Apnea-Hypopnea Index (AHI) score, a sleep score, a therapy score, a flow signal, a pressure signal, a respiration signal, a respiration pattern, a respiration rate, an inspiration amplitude, an expiration amplitude, an inspiration-expiration ratio, a number of events (e.g., apnea events) per hour, a pattern of events, a sleep state and/or sleep stage, a heart rate, a heart rate variability, movement of the user 210, temperature, EEG activity, EMG activity, arousal, snoring, choking, coughing, whistling, wheezing, or any combination thereof.

The one or more sensors 130 can be used to generate, for example, physiological data, audio data, or both. Physiological data generated by one or more of the sensors 130 can be used by the control system 110 to determine the duration of sleep and sleep quality of user 210. For example, a sleep-wake signal associated with the user 210 during the sleep session and one or more sleep-related parameters. The sleep-wake signal can be indicative of one or more sleep states, including sleep, wakefulness, relaxed wakefulness, micro-awakenings, or distinct sleep stages such as a rapid eye movement (REM) stage, a first non-REM stage (often referred to as "N1"), a second non-REM stage (often referred to as "N2"), a third non-REM stage (often referred to as "N3"), or any combination thereof. Methods for determining sleep states and/or sleep stages from physiological data generated by one or more of the sensors, such as sensors 130, are described in, for example, WO 2014/047310, US 2014/0088373, WO 2017/132726, WO 2019/122413, and WO 2019/122414, each of which is hereby incorporated by reference herein in its entirety.

The sleep-wake signal can also be timestamped to determine a time that the user enters the bed, a time that the user exits the bed, a time that the user attempts to fall asleep, etc. The sleep-wake signal can be measured by the one or more sensors 130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory therapy device 122, or any combination thereof during the sleep session.

The event(s) can include snoring, apneas, central apneas, obstructive apneas, mixed apneas, hypopneas, a mouth leak, a mask leak (e.g., from the user interface 124), a restless leg, a sleeping disorder, choking, an increased heart rate, a heart rate variation, labored breathing, an asthma attack, an epileptic episode, a seizure, a fever, a cough, a sneeze, a snore, a gasp, the presence of an illness such as the common cold or the flu, or any combination thereof. In some implementations, mouth leak can include continuous mouth leak, or valve-like mouth leak (i.e. varying over the breath duration) where the lips of a user, typically using a nasal/nasal pillows mask, pop open on expiration. Mouth leak can lead to dryness of the mouth, bad breath, and is sometimes colloquially referred to as "sandpaper mouth."

The one or more sleep-related parameters that can be determined for the user during the sleep session based on the sleep-wake signal include, for example, sleep quality metrics such as a total time in bed, a total sleep time, a sleep onset latency, a wake-after-sleep-onset parameter, a sleep efficiency, a fragmentation index, or any combination thereof.

The data generated by the one or more sensors 130 (e.g., physiological data, flow rate data, pressure data, motion data, acoustic data, etc.) can also be used to determine a respiration signal. The respiration signal is generally indicative of respiration or breathing of the user. The respiration signal can be indicative of a respiration pattern, which can include, for example, a respiration rate, a respiration rate variability, an inspiration amplitude, an expiration amplitude, an inspiration-expiration ratio, and other respiration-related parameters, as well as any combination thereof. In some cases, during a sleep session, the respiration signal can include a number of events per hour (e.g., during sleep), a pattern of events, pressure settings of the respiratory therapy device 122, or any combination thereof. The event(s) can include snoring, apneas (e.g., central apneas, obstructive apneas, mixed apneas, and hypopneas), a mouth leak, a mask leak (e.g., from the user interface 124), a restless leg, a sleeping disorder, choking, an increased heart rate, labored breathing, an asthma attack, an epileptic episode, a seizure, or any combination thereof.

Generally, the sleep session includes any point in time after the user 210 has laid or sat down in the bed 230 (or another area or object on which they intend to sleep), and/or has turned on the respiratory therapy device 122 and/or donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory therapy device 122, and/or gets out of bed 230. In some implementations, the sleep session can include additional periods of time, or can be limited to only some of the above-disclosed time periods. For example, the sleep session can be defined to encompass a period of time beginning when the respiratory therapy device 122 begins supplying the pressurized air to the airway or the user 210, ending when the respiratory therapy device 122 stops supplying the pressurized air to the airway of the user 210, and including some or all of the time points in between, when the user 210 is asleep or awake.

In some cases, a pre-asleep period can be defined as a period of time before a user falls asleep (e.g., before the user enters light sleep, deep sleep, REM sleep), which can include time before and/or after the user has laid or sat down in the bed 230 (or another area or object on which they intend to sleep). In some cases, the personalized entrainment as disclosed herein can be used during this pre-sleep period, although that need not always be the case. In some cases, for example, personalized entrainment can be used during a sleep session (e.g., while the user is asleep or while the user is periodically awake between light sleep, deep sleep, or REM sleep) and/or after a sleep session (e.g., after the user has awoken and decides to stay awake). In some cases, the personalized entrainment as disclosed herein can be used during the pre-sleep period, continue during the sleep session (e.g., in the same or modified form) and/or after the sleep session has ended (e.g., in the same or modified form).

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122, the user interface 124, or the conduit 126. The pressure sensor 132 can be used to determine an air pressure in the respiratory therapy device 122, an air pressure in the conduit 126, an air pressure in the user interface 124, or any combination thereof. The pressure sensor 132 can be, for example, a capacitive sensor, an electromagnetic sensor, an inductive sensor, a resistive sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The flow rate sensor 134 can be used to generate flow rate data associated with the user 210 (FIG. 2) of the respiratory therapy device 122 during the sleep session. Examples of flow rate sensors (such as, for example, the flow rate sensor 134) are described in WO 2012/012835, which is hereby incorporated by reference herein in its entirety. In some implementations, the flow rate sensor 134 is configured to measure a vent flow (e.g., intentional "leak"), an unintentional leak (e.g., mouth leak and/or mask leak), a patient flow (e.g., air into and/or out of lungs), or any combination thereof. In some implementations, the flow rate data can be analyzed to determine cardiogenic oscillations of the user.

The temperature sensor 136 outputs temperature data that can be stored in the memory device 114 and/or analyzed by the processor 112 of the control system 110. In some implementations, the temperature sensor 136 generates temperature data indicative of a core body temperature of the user 210 (FIG. 2), a skin temperature of the user 210, a temperature of the air flowing from the respiratory therapy device 122 and/or through the conduit 126, a temperature of the air in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user 210 during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. In some implementations, the motion sensor 138 alternatively or additionally generates one or more signals representing bodily movement of the user, from which may be obtained a signal representing a sleep state or sleep stage of the user; for example, via a respiratory movement of the user. In some implementations, the motion data from the motion sensor 138 can be used in conjunction with additional data from another sensor 130 to determine the sleep state or sleep stage of the user. In some implementations, the motion data can be used to determine a location, a body position, and/or a change in body position of the user.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory therapy device 122, the user interface 124, the conduit 126, or the user device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 outputs sound waves. In one or more implementations, the sound waves can be audible to a user of the system 100 (e.g., the user 210 of FIG. 2) or inaudible to the user of the system (e.g., ultrasonic sound waves). The speaker 142 can be used, for example, as an alarm clock or to play an alert or message to the user 210 (e.g., in response to an identified body position and/or a change in body position). In some implementations, the speaker 142 can be used to communicate the audio data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465, each of which is hereby incorporated by reference herein in its entirety. In such implementations, the speaker 142 generates or emits sound waves at a predetermined interval and/or frequency and the microphone 140 detects the reflections of the emitted sound waves from the speaker 142. In one or more implementations, the sound waves generated or emitted by the speaker 142 can have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters (e.g., an identified body position and/or a change in body position) and/or respiration-related parameters described in herein such as, for example, a respiration pattern, a respiration signal (from which, e.g., breath morphology may be determined), a respiration rate, an inspiration amplitude, an expiration amplitude, an inspiration-expiration ratio, a number of events per hour, a pattern of events, a sleep state, a sleep stage, or any combination thereof. In this context, a sonar sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO2018/050913 and WO 2020/104465 mentioned above.

In some cases, a microphone 140 and/or speaker 142 can be incorporated into a separate device, such as body-worn device, such as one or a set of earphones or headphones. In some cases, such a device can include other of the one or more sensors 130.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location and/or a body position of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor). In some such implementations, the RF sensor 147 includes a control circuit. The specific format of the RF communication could be Wi-Fi, Bluetooth, etc.

In some implementations, the RF sensor 147 is a part of a mesh system. One example of a mesh system is a Wi-Fi mesh system, which can include mesh nodes, mesh router(s), and mesh gateway(s), each of which can be mobile/movable or fixed. In such implementations, the Wi-Fi mesh system includes a Wi-Fi router and/or a Wi-Fi controller and one or more satellites (e.g., access points), each of which include an RF sensor that the is the same as, or similar to, the RF sensor 147. The Wi-Fi router and satellites continuously communicate with one another using Wi-Fi signals. The Wi-Fi mesh system can be used to generate motion data based on changes in the Wi-Fi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or any combination thereof) that can be stored in the memory device 114. The image data from the camera 150 can be used by the control system 110 to determine one or more of the sleep-related parameters described herein. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or any combination thereof. Further, the image data from the camera 150 can be used to identify a location and/or a body position of the user, to determine chest movement of the user 210, to determine air flow of the mouth and/or nose of the user 210, to determine a time when the user 210 enters the bed 230, and to determine a time when the user 210 exits the bed 230. The camera 150 can also be used to track eye movements, pupil dilation (if one or both of the user 210's eyes are open), blink rate, or any changes during REM sleep.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate pattern, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc. In some cases, the PPG sensor 154 can be a non-contact PPG sensor capable of PPG at a distance. In some cases, a PPG sensor 154 can be used in the determination of a pulse arrival time (PAT). PAT can be a determination of the time interval needed for a pulse wave to travel from the heart to a distal location on the body, such as a finger or other location. In other words, the PAT can be determined by measuring the time interval between the R wave of an ECG and a peak of the PPG. In some cases, baseline changes in the PPG signal can be used to derive a respiratory signal, and thus respiratory information, such as respiratory rate. In some cases, the PPG signal can provide SpO2 data, which can be used in the detection of sleep-related disorders, such as OSA.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein. In some cases, the amplitude and/or morphology changes in the ECG electrical trace can be used to identify a breathing curve, and thus respiratory information, such as a respiratory rate.

In some cases, an ECG signal and/or a PPG signal can be used in concert with a secondary estimate of parasympathetic and/or sympathetic innervation, such as via a galvanic skin response (GSR) sensor. Such signals can be used to identify what actual breathing curve is occurring, and if it has a positive, neutral, or negative impact on the stress level of the individual.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state or sleep stage of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a face mask that covers the nose and mouth of the user 210, the analyte sensor 174 can be positioned within the face mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the face mask (in implementations where the user interface 124 is a face mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory therapy device 122, etc.). Thus, in some implementations, the moisture sensor 176 can be positioned in the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user 210, for example, the air inside the bedroom of the user 210. The moisture sensor 176 can also be used to track the user 210's biometric response to environmental changes.

One or more Light Detection and Ranging (LiDAR) sensors 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor(s) 178 may also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, an orientation sensor, a rain sensor, a soil moisture sensor, a water flow sensor, an alcohol sensor, or any combination thereof.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170, or any combination thereof. For example, the microphone 140 and speaker 142 is integrated in and/or coupled to the user device 170 and the pressure sensor 130 and/or flow rate sensor 132 are integrated in and/or coupled to the respiratory therapy device 122. In some implementations, at least one of the one or more sensors 130 is not coupled to the respiratory therapy device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

The data from the one or more sensors 130 can be analyzed to determine one or more physiological parameters, which can include a respiration signal, a respiration rate, a respiration pattern or morphology, respiration rate variability, an inspiration amplitude, an expiration amplitude, an inspiration-expiration ratio, a length of time between breaths, a time of maximal inspiration, a time of maximal expiration, a forced breath parameter (e.g., distinguishing releasing breath from forced exhalation), an occurrence of one or more events, a number of events per hour, a pattern of events, a sleep state, a sleep stage, an apnea-hypopnea index (AHI), a heart rate, heart rate variability, movement of the user 210, temperature, EEG activity, EMG activity, ECG data, a sympathetic response parameter, a parasympathetic response parameter or any combination thereof. The one or more events can include snoring, apneas, central apneas, obstructive apneas, mixed apneas, hypopneas, an intentional mask leak, an unintentional mask leak, a mouth leak, a cough, a restless leg, a sleeping disorder, choking, an increased heart rate, labored breathing, an asthma attack, an epileptic episode, a seizure, increased blood pressure, or any combination thereof. Many of these physiological parameters are sleep-related parameters, although in some cases the data from the one or more sensors 130 can be analyzed to determine one or more non-physiological parameters, such as non-physiological sleep-related parameters. Non-physiological parameters can also include operational parameters of the respiratory therapy system, including flow rate, pressure, humidity of the pressurized air, speed of motor, etc. Other types of physiological and non-physiological parameters can also be determined, either from the data from the one or more sensors 130, or from other types of data.

The user device 170 (FIG. 1) includes a display device 172. The user device 170 can be, for example, a mobile device such as a smart phone, a tablet, a gaming console, a smart watch, a laptop, or the like. Alternatively, the user device 170 can be an external sensing system, a television (e.g., a smart television) or another smart home device (e.g., a smart speaker(s), optionally with a display, such as Google Home^{™}, Google Nest^{™}, Amazon Echo^{™}, Amazon Echo Show^{™}, Alexa^{™}-enabled devices, etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

The light source 180 is generally used to emit light having an intensity and a wavelength (e.g., color). For example, the light source 180 can emit light having a wavelength between about 380 nm and about 700 nm (e.g., a wavelength in the visible light spectrum). The light source 180 can include, for example, one or more light emitting diodes (LEDs), one or more organic light emitting diodes (OLEDs), a light bulb, a lamp, an incandescent light bulb, a CFL lightbulb, a halogen lightbulb, or any combination thereof. In some implementations, the intensity and/or wavelength (e.g., color) of light emitted from the light source 180 can be modified by the control system 110. The light source 180 can also emit light in a predetermined pattern of emission, such as, for example, continuous emission, pulsed emission, periodic emission of differing intensities (e.g., light emission cycles including a gradual increase in intensity followed by a decrease in intensity), or any combination thereof. Light emitted from the light source 180 can be viewed directly by the user or, alternatively, reflected or refracted prior to reaching the user. In some implementations, the light source 180 includes one or more light pipes.

In some implementations, the light source 180 is physically coupled to or integrated in the respiratory therapy system 120. For example, the light source 180 can be physically coupled to or integrated in the respiratory therapy device 122, the user interface 124, the conduit 126, the display device 128, or any combination thereof. In some implementations, the light source 180 is physically coupled to or integrated in the user device 170 or the activity tracker 190. In other implementations, the light source 180 is separate and distinct from each of the respiratory therapy system 120, the user device 170, and the activity tracker 190. In such implementations, the light source 180 can be positioned to the user 210 (FIG. 2), for example, on the nightstand 240, the bed 230, other furniture, a wall, a ceiling, etc.

The activity tracker 190 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum respiration rate, a respiration rate variability, a heart rate, an average heart rate, a resting heart rate, a maximum heart rate, a heart rate variability, a number of calories burned, blood oxygen saturation level (SpO₂), electrodermal activity (also known as skin conductance or galvanic skin response), a position of the user, a posture of the user, or any combination thereof. The activity tracker 190 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 190 is a wearable device that can be worn by the user, such as a smartwatch, a wristband, a ring, or a patch. For example, referring to FIG. 2, the activity tracker 190 is worn on a wrist of the user 210. The activity tracker 190 can also be coupled to or integrated a garment or clothing that is worn by the user. Alternatively still, the activity tracker 190 can also be coupled to or integrated in (e.g., within the same housing) the user device 170. More generally, the activity tracker 190 can be communicatively coupled with, or physically integrated in (e.g., within a housing), the control system 110, the memory 114, the respiratory therapy system 120, and/or the user device 170.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for generating physiological data and determining a recommended notification or action for the user according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. Thus, various systems can be formed using any portion or portions of the components shown and described herein and/or in combination with one or more other components.

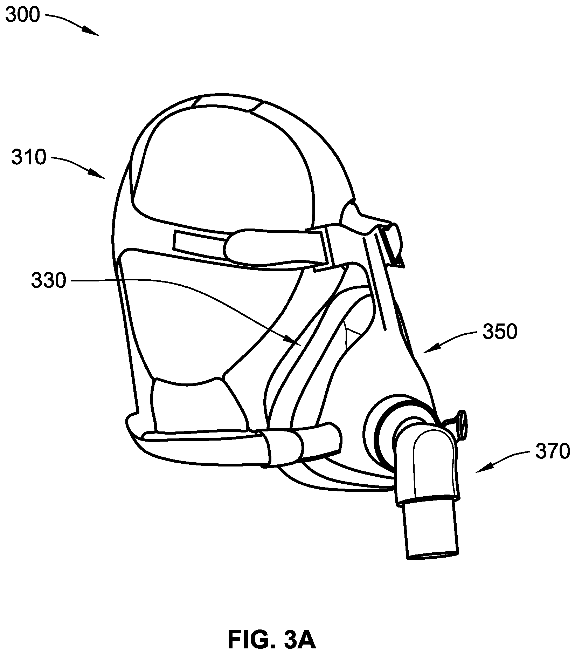

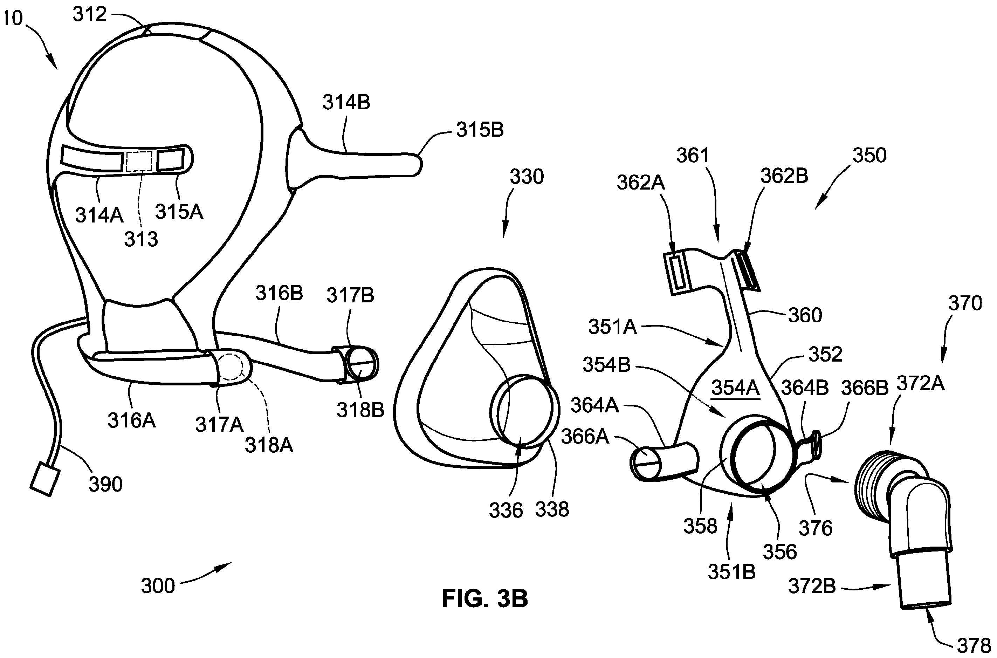

Referring to FIGS. 3A and 3B, a user interface 300, according to some implementations of the present disclosure, is illustrated. The user interface 300 can be the same as, or similar to, the user interface 124 (FIGS. 1 and 2), and can be used with the system 100 described herein. The user interface 300 includes a strap assembly 310, a cushion 330, a frame 350, and a connector 370. The strap assembly 310 is configured to be positioned generally about at least a portion of the user's head when the user wears the user interface 300. The strap assembly 310 can be coupled to the frame 350 and positioned on the user's head such that the user's head is positioned between the strap assembly 310 and the frame 350.

In some implementations, the cushion 330 is positioned between the user's face and the frame 350 to form a seal on the user's face. A first end portion 372A of the connector 370 is coupled to the frame 350, while a second end portion 372B of the connector 370 can be coupled to a conduit (e.g., the conduit 126 shown in FIGS. 1 and 2). In turn, the conduit can be coupled to the air outlet of a respiratory therapy device (e.g., the respiratory therapy device 122 described herein). A blower motor in the respiratory therapy device is operable to flow pressurized air out of the air outlet, to thereby provide pressurized air to the user. The pressurized air can flow from the respiratory therapy device and through the conduit, the connector 370, the frame 350, and the cushion 330, until the air reaches the user's airway through the user's mouth, nose, or both.

The strap assembly 310 is formed from a rear portion 312, a pair of upper straps 314A and 314B, and a pair of lower straps 316A and 316B. The rear portion 312 of the strap assembly is generally positioned behind the user's head when the user wears the user interface 300. The upper straps 314A, 314B and the lower straps 316A, 316B extend from the rear portion 312 toward the front of the user's face. In the illustrated implementation, the rear portion 312 has a circular shape. However, the rear portion 312 may also have other shapes. The rear portion 312, the upper straps 314A, 314B, and the lower straps 316A, 316B can be formed or woven from a generally stretchy or resilient material, such as fabric, elastic, rubber, etc., or any combination of materials. In some implementations, the strap assembly 310 has a hollow interior or channel through which electrical wires or traces may extend, as discussed in further detail below.

The upper straps 314A, 314B and the lower straps 316A, 316B each have first ends originating at the rear portion 312, and second ends that couple to the frame 350. When the user wears the user interface 300, the tension provided by the strap assembly 310 holds the frame 350 to the user's face, thus securing the user interface 300 to the user's head.

In some implementations, a tension sensor can be embedded in one of the straps of the strap assembly. For example, FIG. 3B illustrates a tension sensor 313 embedded in upper strap 314A. The tension sensor 313 is configured to measure tension in the straps of the user interface 124. As discussed, the user interface 124 is generally fasted to the user 210's head using straps that can be tightened using hook and loop fasteners. The tension sensor 313 can sense the tension in the straps, which can then be used to inform and/or instruct the user 210 about the correct fitting of the user interface 124. The tension sensor 313 can be integrated into yarn, fiber, wire, carbon fiber, warps, webs, etc. As the tension in the strap increases or decreases, the sensor element of the tension sensor 313 is deflected, causing a change in the voltage of an output signal. The tension sensor 313 can have high elasticity and low resistance, and the ability to be washed. In some implementations, the tension sensor 313 measures the diameter of an inflatable body by the principles of respiratory inductance plethysmography. The tension sensor 313 can also be an electric impedance plethysmography sensor, a magnetometer, a strain gauge sensor, or be made of piezo-resistive material displacement sensor.

The frame 350 is generally formed from a body 352 that defines a first surface 354A and a second opposing surface 354B. When the user wears the user interface 300, the first surface 354A faces away from the user's face, while the second surface 354B faces toward the user's face. The frame also defines an annular aperture 356 into which the cushion 330 and the connector 370 can be inserted, to thereby physically couple the cushion 330 and the connector 370 to the frame 350.

The cushion 330 can be coupled to the inside of the frame 350 adjacent to the second surface 354B, such that the cushion 330 is positioned between the user's face and the frame 350. The cushion 330 can be made from the same or similar material as the cushion of user interface 124, for example, a conformal material that aids in forming an air-tight seal with the user's face. The cushion 330 defines an aperture 336, and includes an annular projection 338 extending from the cushion 330 about the aperture 336 of the cushion. The annular projection 338 is inserted into the annular aperture 356 of the frame 350, such that the annular aperture 336 of the cushion 330 overlaps with the annular aperture 356 of the frame 350. In some implementations, the annular projection 338 of the cushion 330 is releasbly secured to the body 352 of the frame 350 via a friction fit between the annular projection 338 and the body 352 around the annular aperture 356.

In other implementations, the annular projection 338 and the frame 350 can have mating features that mate with each other to secure the cushion 330 to the frame 350. For example, the annular projection 338 of the cushion 330 may include an outwardly-extending peripheral flange, and the body 352 of the frame 350 can include a corresponding inwardly-extending peripheral flange about the annular aperture 356. When the annular projection 338 of the cushion 330 is inserted into the annular aperture 356 of the frame 350, the peripheral flanges can slide or snap past each other, to thereby secure the cushion 330 to the frame 350. In additional implementations, the cushion 330 is held in place by the tension provided by the strap assembly 310, and is not physically coupled to the frame 350. In still other implementations, the cushion 330 and the frame 350 can be formed as a single integral piece.

The connector 370 can be coupled to the opposite side of the frame 350 in a similar manner to the cushion 330. The first end portion 372A of the connector 370 has a generally cylindrical shape and can be inserted into the annular aperture 356 of the frame 350, such that a hollow interior 376 of the end portion 372A overlaps with the annular aperture 356, and the aperture 336 of the cushion 330. The opposing second end portion 372B of the connector 370 is then coupled to the conduit, such that the user's face (including the user's mouth and/or nose) is in fluid communication with the conduit through the cushion 330, the frame 350, and the connector 370.

The first end portion 372A of the connector 370 is generally annular-shaped, and fits into the annular aperture 356 of the frame 350. The frame 350 also includes an annular projection 358 that extends from the second surface 354B of the frame 350 and is formed about the annular aperture 356. When the first end portion 372A is inserted into the annular aperture 356 of the frame 350, an inner surface of the annular projection 358 overlaps with an outer surface of the first end portion 372AA of the connector 370.

In some implementations, a friction fit between the annular projection 358 and the first end portion 372A secures the connector 370 to the frame 350. In other implementations, the connector 370 can include a fastener configured to secure the connector 370 to the frame 350 (e.g., via a threaded connection). In one example, the annular projection 358 has an outwardly-extending peripheral flange, and the fastener is one or more deflectable latches formed on the first end portion 372A of the connector 370. As the first end portion 372A slides is inserted within the annular projection 358, the deflectable latch slides over the peripheral flange such that the deflectable latch is positioned outside of the annular projection 358. As the deflectable latch passes by the peripheral flange, the peripheral flange pushes the deflectable latch away from the annular projection 358. The deflectable latch then returns to its original position, such that the connector 370 cannot be removed from the frame 350 without manually deflecting the deflectable latch away from the annular projection 358.

The frame 350 includes a T-shaped extension strip 360 extending upward from an upper end 351A of the body 352. In some implementations, the extension strip 360 is integrally formed with the body 352. In other implementations, the extension strip 360 is a separate component that is coupled to the body 352. When the user wears the user interface 300, the extension strip 360 generally extends up to the user's forehead. In some implementations, the extension strip 360 includes a cooling portion or mechanism that contacts and cools the user 210's forehead, which can help users with insomnia fall asleep.

The lower straps 316A, 316B extend toward the frame 350 from the rear portion 312 of the strap assembly 310, and are coupled to opposite sides of a lower end 351B of the body 352. The upper straps 314A, 314B extend toward the frame 350 from the rear portion 312 of the strap assembly 310, and are coupled to opposite sides of the upper end 361 extension strip 360 (e.g., the generally horizontal "cross" of the T). The frame 350 can include a variety of different strap attachment points to couple with the upper straps 314A, 314B and the lower straps 316A, 316B.

One type of strap attachment point is shown in the extension strip 360. The upper end 361 of the extension strip 360 includes two apertures 362A, 362B. These apertures can be integrally formed in the extension strip 360 itself, or may be formed as part of a separate component or piece that is coupled to the extension strip 360. The apertures 362A, 362B are shaped to allow the ends 315A, 315B of the upper straps 314A, 314B to be inserted through the apertures 362A, 362B. The ends 315A, 315B can then loop back and fasten to remainder of the upper straps 314A, 314B via any suitable mechanism, such as hook and loop fasteners, adhesive, etc. The upper straps 314A, 314B are thus secured to the extension strip 360 of the frame 350.

The frame 350 is shown with a different type of strap attachment point used to couple the lower straps 316A, 316B to the frame 350. The frame 350 includes two lateral strips 364A, 364B extending away from opposite ends of the lower end 351B of the body 352. The first end of each lateral strip 364A, 364B is coupled to the body 352, and a corresponding magnet 366A, 366B is disposed at the second end of each lateral strip 364A, 364B. A magnet 318A is coupled to end 317A of lower strap 316A, while a magnet 318B is coupled to end 317B of lower strap 316B. Magnet 318A can be secured to magnet 366A via magnetic attraction, while magnet 318B can be secured to magnet 366B via magnetic attraction, to thereby couple the lower straps 316A, 316B to the body 352 of the frame 350.

In some implementations, the frame 350 does not include the extension strip 360, and the upper straps 314A, 314B are instead coupled to the frame, above the lateral strips 364A, 364B. The upper straps 314A, 314B in these implementations extend past the user 210's temples and around to the rear of the user 210's head. The frame 350 may include upper lateral strips which the upper straps 314A, 314B are coupled to.

The user interface 300 can also include one or more sensors 390. While FIG. 3B generally only shows a single sensor, any number of sensors can be coupled to the strap assembly 310. In some implementation, the one or more sensors 390 are coupled to the strap assembly 310, and are configured to abut a target area of the user when the user wears the user interface 300. The target area could be the user's forehead, temple, throat, neck, ear, etc. In other implementations, the one or more sensor 390 are not coupled to the strap assembly 310, but are instead located at other positions within the user interface 300, such as within the connector 370. The sensors 390 can be any one or more of the sensors 130 described herein with respect to FIG. 1, and can additionally or alternatively include other types of sensors.

In some implementations, the one or more sensors 390 includes one or more contact sensors that contact the target area of the user. For example, the one or more sensors 390 can include an electroencephalography (EEG) sensor, an electrocardiogram (ECG) sensor, an electromyography (EMG) sensor, an electrooculography (EOG) sensor, an acoustic sensor, a peripheral oxygen saturation (SpO₂) sensor, a galvanic skin response (GSR) sensor, or any combination thereof.

In some implementations, the one or more sensors 390 additionally or alternatively includes one or more non-contact sensors, which can include a carbon dioxide (CO₂) sensor (to measure CO₂ concentration), an oxygen (O₂) sensor (to measure O₂ concentration), a pressure sensor, a temperature sensor, a motion sensor, a microphone, an acoustic sensor, a flow sensor, a tension sensor, or any combination thereof.

In still further implementations, one or more sensors 390 includes one or more contact sensors that are configured to contact the target area of the user, and one or more non-contact sensors. In some of these implementations, the non-contact sensor is not coupled to the strap assembly 310, but is instead disposed in the cushion 330, the frame 350, or the connector 370. Moreover, the user interface 300 can include multiple non-contact sensors disposed in any combination of these locations.

Generally, the one or more sensors 390 of the user interface 300 need to be electrically connected to a control system and a memory device of the respiratory therapy system (such as control system 110 and memory device 114 of the system 100) in order to transmit data to the control system and memory device. These data can be used to modify the operation of the respirator device, and can also be used for other purposes. In order send the data from the one or more sensors 390 to the control system and memory device, the one or more sensors 390 can be electrically connected to various parts of the user interface 300, including the frame 350 and the connector 370. Data from the one or more sensors 390 can be transmitted using the electrical connection between the one or more sensors 390, the frame 350, and the connector 370. Thus, wherever the one or more sensors 390 are located in the user interface 300, the one or more sensors 390 need to be able to be electrically connected to the control system and the memory device.

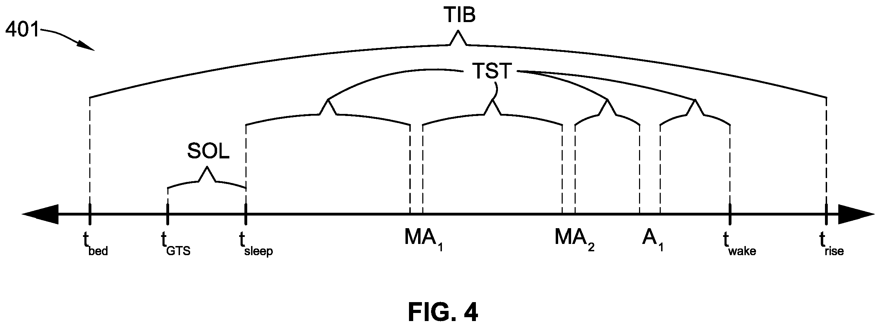

As used herein, a sleep session can be defined in a number of ways based on, for example, an initial start time and an end time. Referring to FIG. 4, an exemplary timeline 400 for a sleep session is illustrated. The timeline 400 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (trise).

In some implementations, a sleep session is a duration where the user is asleep. In such implementations, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) a user-selectable element that is displayed on the display device 172 of the user device 170 (FIG. 1) to manually initiate or terminate the sleep session.

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 4 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (t_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 401 of FIG. 4, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (t_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

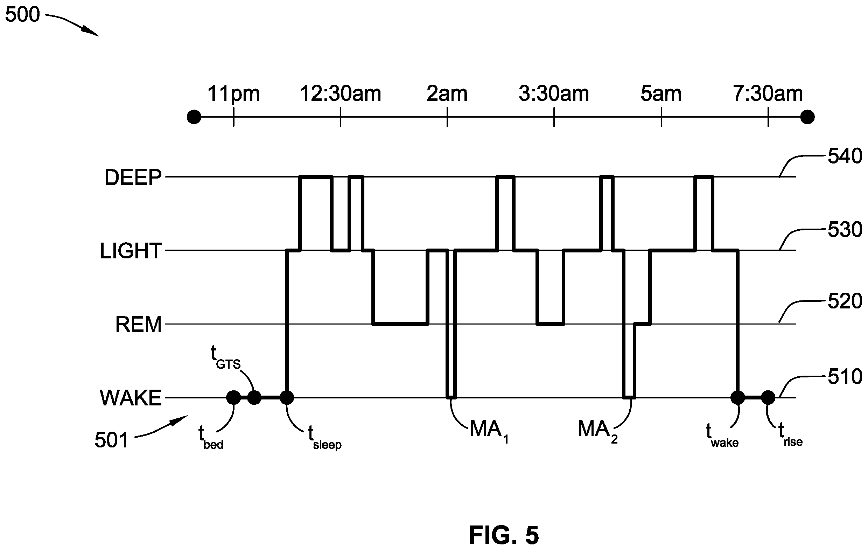

Referring to FIG. 5, an exemplary hypnogram 500 corresponding to the timeline 401 (FIG. 4), according to some implementations, is illustrated. As shown, the hypnogram 500 includes a sleep-wake signal 501, a wakefulness stage axis 510, a REM stage axis 520, a light sleep stage axis 530, and a deep sleep stage axis 540. The intersection between the sleep-wake signal 501 and one of the axes 510-540 is indicative of the sleep stage at any given time during the sleep session.

The sleep-wake signal 501 can be generated based on physiological data associated with the user (e.g., generated by one or more of the sensors 130 (FIG. 1) described herein). The sleep-wake signal can be indicative of one or more sleep states or stages, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 500 is shown in FIG. 5 as including the light sleep stage axis 530 and the deep sleep stage axis 540, in some implementations, the hypnogram 500 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 500 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (t_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 5), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

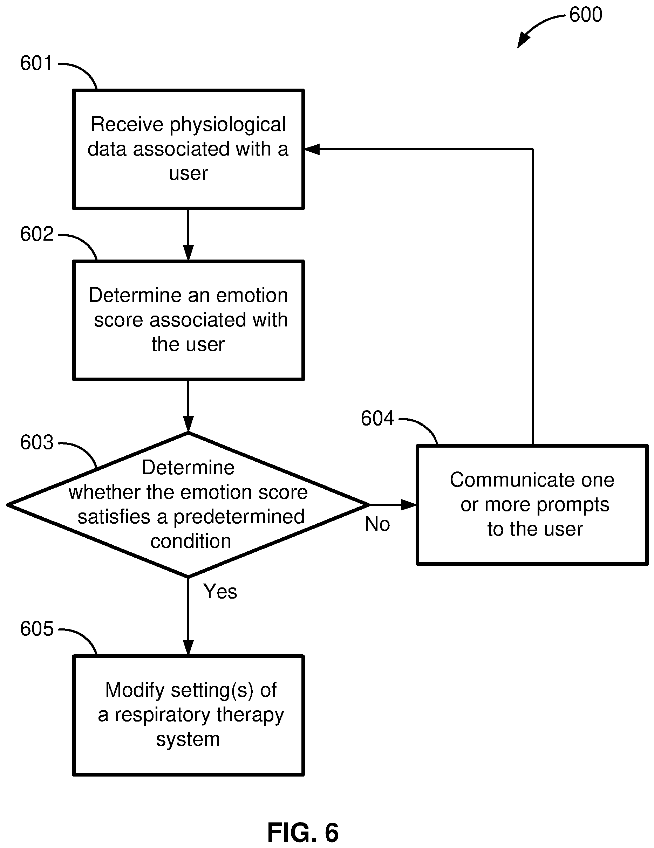

Referring to FIG. 6, a method 600 for aiding a user according to some implementations of the present disclosure is illustrated. One or more steps or aspects of the method 600 can be implemented using any portion or aspect of the system 100 described herein.

Step 601 of the method 600 includes receiving physiological data associated with a user. The physiological data can be generated by, and received from, one or more of the sensors 130 (FIG. 1) described herein. The received physiological data can indicative of one or more physiological parameters such as, for example, movement, heart rate, heart rate variability, cardiac waveform, respiration rate, respiration rate variability, respiration depth, a tidal volume, an inspiration amplitude, an inspiration duration, an expiration amplitude, an expiration duration, an inspiration-expiration ratio, perspiration, temperature (e.g., ambient temperature, body temperature, core body temperature, surface temperature, etc.), blood oxygenation, photoplethysmography, pulse transit time, blood pressure, peripheral arterial tone, cardiogenic oscillations, a galvanic skin response, a sympathetic nervous system response, a pulse transit time, a trend associated with the heart rate, a trend associated with the respiration rate, a trend associated with the galvanic skin response, or any combination thereof. The physiological data can be received from at least one of the one or more sensors 130 by, for example, the electronic interface 119 and/or the user device 170 described herein, and stored in the memory 114 (FIG. 1). The physiological data can be received by the electronic interface 119 or the user device 170 from at least one of the one or more sensors 130 either directly or indirectly (e.g., with one or more intermediaries). The physiological data can include voice, which can be an indicator of stress/anxiety and can be received by the microphone 140. The microphone 140 can be integrated in a microphone sensor (e.g., an Amazon^{®} Halo device).

In some implementations, the physiological data is generated during at least a portion of a sleep session. For example, a first portion of the physiological data for the sleep session can be associated with a first day (e.g., a Monday) before or when the user begins the sleep session. If the first sleep session extends into the next day (e.g., a Tuesday) before ending, the physiological data will also be associated with a portion of the second day. In this example, the first portion of the physiological data can be associated with any portion of the sleep session (e.g., 10% of the sleep session, 25% of the sleep session, 50% of the sleep session, 100% of the sleep session, etc.).

In some implementations, the physiological data received in step 601 is generated or obtained (e.g., by one or more of the sensors 130) before the user wears or dons the user interface 124. In other implementations, the physiological data is generated before and after the user wears or dons the user interface 124. In such implementations, the physiological data can be generated by a first one of the one or more sensors 130 before the user wears the user interface 124 (e.g., a sensor that is physically coupled to or integrated in the user device 170 or the activity tracker 190) and a second one of the one or more sensors 130 after the user wears the user interface 124 (e.g., a sensor that is physically coupled to or integrated in the respiratory therapy system 120).

Step 602 of the method 600 includes determining an emotion score associated with the user based at least in part on the physiological data. Generally, the emotion score is indicative of anxiety or stress currently being experienced by the user. For example, the user may experience anxiety, stress, apprehension, discomfort, etc. when wearing the user interface 124 of the respiratory therapy system 120, especially when the user is not accustomed to wearing the user interface 124. Higher levels of stress, anxiety, discomfort, etc. can make it more difficult for the user to fall asleep and/or may prompt the user to abandon using the respiratory therapy system 120. A quantification of the stress or anxiety of the user via the emotion score can be used to suggest or recommend actions or activities to reduce the anxiety or stress and aid the user in falling asleep and becoming comfortable with using the respiratory therapy system 120.

An emotion score can be an absolute value or can be a relative value. The emotion score can be, for example, a numerical value that is on a predetermined scale (e.g., between 1-10, between 1-100, etc.), a letter grade (e.g., A, B, C, D, or F), or a descriptor (e.g., high, low, medium, poor, normal, abnormal, fair, good, excellent, average, below average, above average, needs improvement, satisfactory, etc.). According to the invention, the emotion score is determined relative to a previous emotion score (e.g., the emotion score is better than a prior emotion score (e.g., an emotion score for the prior day), the emotion score is worse than a prior emotion score, the emotions score is the same as a prior emotion score, etc.) or a baseline emotion score (e.g., the emotion score is 50% greater than the baseline emotion score, the emotion score is equal to the baseline emotion score, etc.). In some implementations, the prior emotion score can be a target emotion score. Examples of target emotion scores include a score at which the user was able to fall asleep, a score at which the user had a short sleep onset latency, etc.

In some implementations, the quantification of the stress or anxiety of the user can be based on a change from the baseline emotion score. The quantification can take the form of a difference (relative or absolute) from the target emotion score.

In some implementations, the physiological data generated before the user wears or dons the user interface 124 at step 601 is representative of the baseline emotion score. In some implementations, the baseline emotion score can be determined from the physiological data at times when the user would be expected to be relaxed (e.g. when the user is about to fall asleep, when the user has just fallen asleep, etc.). The physiological data indicative of sleep stages can be used to determine the different times. The baseline emotion score can be determined from wearable electronics (e.g., the activity tracker 190) worn by the user, subjective information obtained by the user, etc. In some implementations, one or more prompts can be provided to the user to ascertain the baseline emotion score.

In some implementations, step 602 includes determining one or more physiological parameters associated with the user, such as, for example, movement, a respiration rate, heart rate, heart rate variability, cardiac waveform, respiration rate, respiration rate variability, respiration depth, a tidal volume, an inspiration amplitude, an inspiration duration, an expiration amplitude, an expiration duration, an inspiration-expiration ratio, perspiration, temperature (e.g., ambient temperature, body temperature, core body temperature, surface temperature, etc.), blood oxygenation, photoplethysmography (e.g., which can be used to measure SpO₂, peripheral perfusion, peripheral arterial tone, pulse-rate, other cardiac-related parameters, etc.), pulse transmit time, blood pressure, cardiogenic oscillations, a galvanic skin response, a sympathetic nervous system response, a pulse transit time, a trend associated with the heart rate, a trend associated with the galvanic skin response, a trend associated with the respiration rate, or any combination thereof.

These physiological parameters can be indicative of emotion or anxiety of the user. For example, hyperventilation, increased respiration rate (e.g., relative to a baseline associated with the user, a baseline for a group of users, normative values, etc.), decrease heart rate variability (e.g., relative to a baseline associated with the user, a baseline for a group of users, normative values, etc.), cardiac arrhythmias, heart rate, and blood pressure (e.g., adjusting for whether the user is hypertensive or non-hypertensive, nocturnal dips in blood pressure, etc.) can be indicative of an increased anxiety level. Conversely, increased heart rate variability (e.g., relative to a baseline associated with the user, a baseline for a group of users, normative values, etc.) can be indicative of a more relaxed state. The emotion score can be determined, at least in part, by scaling or standardizing one or more of the physiological parameters based on previously recorded physiological parameters for the user, previously recorded physiological parameters for a plurality of other users, or both, that are stored in the user profile described above. Alternatively, the emotion score can be determined by scaling the associated physiological parameter(s) with a desired or target value for the parameter(s).

The physiological parameters can be individual-specific. The physiological parameters can be monitored to determine whether the physiological parameters are within range. For example, a typical adult respiration rate may be between 12-20 breaths per minute (bpm), and respiration rates greater than 25 bpm may indicate anxiety/stress. Similarly, a typical adult heart rate may be 60-100 beats per minute, and heart rates greater than 120bpm may indicate anxiety/stress. Increase in body temperature can indicate anxiety/stress. Furthermore, if a value of a physiological parameter changes after a stimulus such as a beginning of therapy, or increases in therapy pressure, but then returns to a baseline value relatively quickly (e.g. within 5-10 minutes, or other predetermined period), then an increased emotion score can be acceptable. The increased emotion score is acceptable because slight increase is temporary, thus no (or a minimal number of prompts) is needed to adjust the emotion score.

In some implementations, step 602 further includes receiving subjective feedback from the user and determining the emotion score based at least in part on the received subjective feedback. The subjective feedback can include, for example, self-reported user feedback indicative of a current stress or anxiety level of the user in the form of, for example, a descriptive indicator (e.g., high, low, medium, not sure), a numerical value (e.g., on a scale of 1 to 10, where 10 is very stressed and 0 is not stressed at all), etc. Stress or anxiety levels of the user may be based on or may correspond to the Likert scale. The subjective feedback can be received, for example, via the user device 170. In such implementations, step 602 can include communicating one or more prompts to the user to solicit the subjective feedback (e.g., via the display device 172 of the user device 170).

In some implementations, step 602 includes determining the emotion score based at least in part on demographic information associated with the user. The demographic information can include, for information, indicative of an age of the user, a gender of the user, a weight of the user, a body mass index (BMI) of the user, a height of the user, a race of the user, a relationship or marital status of the user, a family history of insomnia, an employment status of the user, an educational status of the user, a socioeconomic status of the user, or any combination thereof. The demographic information can also include medical information associated with the user, such as, for example, including indicative of one or more medical conditions associated with the user, medication usage by the user, or both. The demographic information can be received by and stored in the memory 114 (FIG. 1). The demographic information can be provided manually by the user, for example, via the user device 170 (e.g., via a questionnaire or survey presented through the display device 172). Alternatively, the demographic information can be collected automatically from one or more data sources associated with the user (e.g., medical records). Demographic information can be a useful input to determining the emotion score since certain physiological parameters (e.g., heart rate) can change as a function of age, medical conditions, etc.

Step 603 of the method 600 includes determining whether the first emotion score satisfies a predetermined condition. Generally, the predetermined condition is indicative of the anxiety or stress of the user being at an acceptable level (e.g., such that the user can fall asleep, begin using the respiratory therapy system 120, increase the pressure of the air supplied to the user via the user interface 124, etc.). In other words, when the emotion score satisfies the predetermined condition, the user is sufficiently relaxed such that the user can fall asleep and/or the respiratory therapy system 120 can be used and/or the pressure of the air supplied to the user via the user interface 124 can be increased. The acceptable level can be learned from the user over a number of sleep sessions and/or over a calibration period when the user begins to use the respiratory therapy system 100. The acceptable level can be learned from a population of users (e.g., a cohort), normative values, etc.

For example, if the emotion score is a numerical value where a higher numerical value indicates higher anxiety or stress, the predetermined condition can be a numerical value that is indicative of anxiety or stress of the user being at an acceptable level. In this example, the emotion score satisfies the predetermined condition if the emotion score is equal to or less than the predetermined condition.

In some implementations, the predetermined condition is determined based at least in part on previously recorded physiological data associated with the user. In such implementations, the predetermined condition can be determined using a machine learning algorithm. The machine learning algorithm can be trained (e.g., using supervised or unsupervised training techniques) with previously recorded physiological data associated with the user such that the machine learning algorithm is configured to determine the predetermined condition. In such implementations, previously recorded physiological data can include corresponding data relating the user's ability to fall asleep, such as, for example, sleep onset latency, wake-after-sleep onset, sleep efficiency, fragmentation index, time to go to bed, total time in bed, total sleep time, or any combination thereof. The previously recorded data for training the machine learning algorithm can also include subjective feedback from the user, as described herein. The previously recorded data for training the machine learning algorithm can be data from a population of users or a cohort of users residing in a similar demographic as the user. Data from the cohort of users can be used for initial settings prior to learning from the user's physiological data.

In some implementations, the method 600 includes communicating one or more indications of the determined emotion score to the user (e.g., in the form of a report). For example, an indication of the determined emotion score can be communicated to the user via the user device 170. The indication can be communicated to the user before, during, or after the sleep session. Additionally or alternatively, an indication of the determined emotion score can be communicated to a third party (e.g., a medical provider, a physician, etc.). Additionally, the method 600 can further include communicating one or more indications of any of the sleep-related parameters described herein to the user. For example, an indication of a sleep-related parameter can be communicated to the user via the user device 170 before, during, or after the sleep session. Emotion and/or anxiety, subject to the emotion score, can result in insomnia (e.g., anxiety-induced insomnia). Thus, tracking emotion via scoring can help explain an insomniac condition of the user.

Step 604 of the method 600 includes causing one or more prompts to be communicated to the user to aid in modifying the emotion score in response to determining that the determined emotion score (step 602) does not satisfy the predetermined condition (step 603). The one or more prompts are generally used to aid in modifying (e.g., improving) the determined first emotion score. The one or more prompts can include one or more visual prompts, one or more audio prompts, one or more tactile prompts, or any combination thereof. The one more visual prompts can be communicated to the user, for example, via the display device 128 of the respiratory therapy system 120, via the display device 172 of the user device 170, via the light source 180, the activity tracker 190, or any combination thereof. The one or more audio and/or tactile prompts can be communicated to the user via a transducer, such as the speaker 142, which can be physically coupled to or integrated in, for example, the respiratory therapy device 122, the user device 170, or the activity tracker 190. The one or more audio and/or tactile prompts can be conducted by, for example, the user interface 124 and/or the conduit 126, and/or by bone conduction, to the user. The one or more audio and/or tactile prompts can be communicated in a predetermined pattern, such as continuous, pulsed, periodic communication at differing intensities, or any combination thereof.

In some implementations, step 604 includes causing one or more visual prompts to be communicated to the user via the light source 180 to aid in modifying the emotion score. As described herein, in some examples, the light source 180 can be physically coupled to or integrated in the respiratory therapy system 120. Alternatively, the light source 180 can be physically separate and distinct from the respiratory therapy system 120 (e.g., physically integrated or coupled to the user device 170, the activity tracker 190, etc.). In such implementations, step 604 includes causing the light source 180 to emit light having a predetermined color, a predetermined intensity, a predetermined frequency (e.g., light pulses), or any combination thereof. The one or more prompts can include, for example, changing a color of the light emitted from the light source 180, changing an intensity of the light emitted from the light source 180, or both.

As described above, step 602 can include determining, among other things, a respiration rate associated with the user. Step 604 can include causing the light source 180 to emit light pulses at a predetermined frequency to aid in modifying (e.g., decreasing) the respiration rate. For example, each light pulse can signal to the user to breath in (e.g., at the beginning of the pulse) and/or breath out (e.g., at the end of the pulse) to aid in modifying the respiration rate, and in turn the emotion score. In some implementations, based on the detected respiration rate of the user, the pattern of light pulses could initially mirror the user's respiration rate and then gradually change (e.g., decrease) in order to encourage the user's respiration rate to change to a desired rate, and in turn a desired emotion score. Alternatively, step 602 can include communicating one or more prompts to perform a breathing exercise to the user to aid in modifying the respiration rate, and in turn the emotion score. In some implementations, the target breathing pattern can be overlaid on the pressure settings of the respiratory therapy system by, for example, introducing a modulation on the motor RPM as a target pattern to provide a gentle, guided breathing, without performing ventilation. In such implementations, the modulation can be done at low pressures (e.g., 10% of the maximum pressure setting, 25% of the maximum pressure setting, 50% of the maximum pressure setting, etc.), before or together with the pressure ramp, which typically occurs at the beginning of a therapy session. The pressure ramp or target pressure can then continue once emotion score satisfies a predetermined condition and/or the user has fallen into sustained sleep.

As described above, in some examples, the user is wearing the user interface 124 when the first physiological data (step 601) is being generated. Thus, in some implementations, the one or more prompts that are communicated to the user in step 604 can include a prompt or recommendation to remove the user interface 124 to aid in modifying the emotion score. For example, the user can be prompted to remove the user interface 124 for a predetermined duration (e.g., 1 minute, 5 minutes, 10 minutes, etc.). The user can further be prompted to perform breathing exercises once the interface 124 has been removed to further aid in modifying the emotion score.

In some implementations, the one or more prompts that are communicated to the user include one or more instructions to perform an activity. The instructions to perform an activity can be communicated visually (e.g., via alphanumeric text displayed on the display device 172, images or photos displayed on the display device 172, etc.) and/or via audio (e.g., via the user device 170 or speaker 142). The instructions can include, for example, instructions how to assemble the various components of the user interface 300 (FIGS. 3A and 3B) before wearing the user interface 300. These assembly instructions for the user interface 300 can include information as to the function or purpose of each component and can generally aid in modifying the emotion score.

In some implementations, one or more prompts that are communicated to the user in step 604 can include media content that further aids in modifying the emotions score. For example, the media content can include audio (e.g., music, an e-book, etc.), video (e.g., a television show, movie), photos, etc. to aid in modifying the emotion score. The media content can be delivered to the user, for example, via the user device 170 or another device (e.g., a television, laptop, tablet, etc.).

In some implementations, step 604 includes selecting the one or more prompts to be communicated to the user. For example, the one or prompts can be selected based at least in part on previously recorded data associated with the user (e.g., based on the user's prior responses to the one or more prompts). In such implementations, a machine learning algorithm can be trained with previously recorded physiological data and/or emotion score associated with the user recorded before and/or after one or more prompts have been communicated to the user. Thus, selecting the one or more prompts can include using the machine learning algorithm that is trained (e.g., using supervised or unsupervised techniques) to receive as an input current physiological data and/or an emotion score associated with the user and determine as an output one or more prompts to communicate to the user to aid in modifying the determined emotion score. In this way, the present method can, for example, identify the one or more prompts that allows the user's emotion score to achieve the predetermined condition. In particular implementations, the present method can identify the one or more prompts such as instructions to breathe deeply, alter inspiration-expiration ratio, etc., that best reduces heart rate, increases heart rate variability, and leads to faster transition to light sleep N1.

As described above, step 604 is performed in response to determining in step 603 that the emotion score does not satisfy the predetermined condition. After the one or more prompts are communicated to the user in step 604, steps 601 - 604 can be repeated one or more times to aid in modifying the emotion score until the emotion score satisfies the predetermined condition. In response to determining that the emotion score satisfies the predetermined condition, the method 600 proceeds to step 605.

Step 605 of the method 600 includes modifying one or more settings of the respiratory therapy system responsive to determining that the first emotion score satisfies the predetermined condition (step 603). Alternatively, or in addition, the modifying one or more settings of the respiratory therapy system is responsive to determining the sleep state (e.g. awake, asleep, etc.) and/or sleep stage (e.g. N1, N2, etc.) of the user For example, step 605 can include modifying a pressure setting of the respiratory therapy device 122 (e.g., turn on/off the respiratory therapy device 122 for delivering pressurized air, increasing the pressure, decreasing the pressure, modifying a maximum pressure setting, modifying a minimum pressure setting, modifying the ramp duration, etc.), modifying conduit temperature, modifying humidification of the pressurized air, modifying comfort settings such as expiratory pressure relief (EPR), or any combination thereof.

In some implementations, step 605 includes causing the respiratory therapy device 122 of the respiratory therapy system 120 to supply pressurized air. In such implementations, the respiratory therapy device 122 does not deliver pressurized air, or delivers pressurized air at a low pressure, prior to determining that the emotion score satisfies the predetermined condition. In other words, in such implementations, step 605 includes actuating or turning on the respiratory therapy device 122.

In some implementations, the respiratory therapy device 122 of the respiratory therapy system 120 supplies pressurized air at a first predetermined pressure prior to determining that the first emotion score satisfies the predetermined condition (step 603). In such implementations, step 605 can include causing the respiratory therapy device 122 of the respiratory therapy system 120 to supply pressurized air at a second predetermined pressure that is different than the first predetermined pressure. In some implementations, the second predetermined pressure is greater than the first predetermined pressure. For example, if the determined first emotion score is below a predetermined threshold (e.g., that is indicative of the user being relaxed), the second predetermined pressure can be greater than the first predetermined pressure. In this example, the first predetermined pressure can be gradually increased until reaching the second predetermined pressure. In other implementations, the second predetermined pressure is less than the first predetermined pressure. For example, if the determined first emotion exceeds a predetermined threshold (e.g., the user is experiencing anxiety while wearing the interface 124 and receiving pressurized air), the first predetermined pressure can be reduced to the second predetermined pressure to further aid in modifying the emotion score.

In some implementations, step 605 includes modifying one or more settings of one or more devices that are external to the respiratory therapy system 120. For example, step 605 can include modifying one or more settings of the user device 170 (e.g., the content displayed on the display device 172) and/or one or more settings of the activity tracker 190. As another example, step 605 can include modifying one or more settings of one or more Internet of Things (IoT) devices, such as, for example, a smart appliance (e.g., television), a smart thermostat or HVAC system, smart lighting, etc. The user's response (e.g., change in emotion score) to the modified settings can be used to determine one or more prompts to communicate to the user to aid in modifying the emotion score in the future.

In some implementations, the method 600 includes determining a therapy recommendation for the user based at least in part on the first emotion score. In such implementations, the method 600 can include communicating an indication of the recommendation to the user, a third party (e.g., medical provider), or both. The therapy recommendation can include, for example, a recommendation to modify a type of user interface for the respiratory therapy system (e.g., a full face mask, a nasal pillows mask, a nasal mask, etc.). The therapy recommendation can also include a medication recommendation, a recommendation to cease using the respiratory therapy system, a recommendation to use an alternative medical device (e.g., a mandibular repositioning device, a neurostimulatory device, etc.), or any combination thereof.

As described above, steps 601 to 604 can be repeated one or more times until the emotion scores satisfies the predetermined condition. Additionally, steps 601 to 604 can be repeated one or more times after the one or more settings of the respiratory therapy system have been modified in step 605. For example, if the user falls asleep using the user interface 124 but wakes up in the middle of the sleep session, the emotion score is updated based on the physiological data. If the emotion score does not satisfy the predetermined threshold, one or more settings of the respiratory therapy system can be further modified (e.g., turned off, decreasing the pressure, prompting the user to take off the user interface 124, etc.). One advantage to some implementations of the present disclosure is that should be user not comply with their therapy prescription, a physician/medical device supplier can better understand reasons for the non-compliance. For example, the physician/medical device supplier can determine that the user is unable to use the respiratory therapy device 122 comfortably and are stressed, rather than being lazy or forgetful for example.

While described above in reference to one sleep session, one or more steps of the method 600 can be repeated one or more times for additional sleep sessions (e.g., 2 sleep sessions, 3 sleep sessions, 10 sleep sessions, 100 sleep sessions, 500 sleep sessions, etc.). If it is detected that the user is not anxious over a number of therapy sessions since, for example, the respiratory therapy device 122 is operating at a sub-optimal therapy/low pressure setting, therapy may be adjusted to closer to the optimal (e.g., prescribed) therapy setting. Further while steps 601 to 605 have been shown and described herein in a particular order, more generally, steps 601 to 605 can be performed in any suitable order and/or simultaneously.

One or more elements or aspects or steps, or any portion(s) thereof, from one or more of any of claims 1-62 below can be combined with one or more elements or aspects or steps, or any portion(s) thereof, from one or more of any of the other claims 1-62 or combinations thereof, to form one or more additional implementations and/or claims of the present disclosure.

While the present disclosure has been described with reference to one or more particular embodiments or implementations, those skilled in the art will recognize that many changes may be made thereto without departing from the scope of the present disclosure Each of these implementations and obvious variations thereof is contemplated as falling within the scope of the present disclosure.

## Claims

1. A system comprising:
a respiratory therapy system (120);
a memory (114) having stored thereon machine-readable instructions; and
a control system (110) including one or more processors (112) configured to execute the machine-readable instructions to:
receive (601) first physiological data associated with a user;
determine (602) a first emotion score associated with the user based at least in part on the first physiological data, the first emotion score being determined relative to a prior emotion score at which the user was able to fall asleep; and
responsive to determining that the first emotion score satisfies a predetermined condition, determine (605) a modification of one or more settings of the respiratory therapy system (120).

2. The system of claim 1, wherein the control system (110) is further configured to execute machine-readable instructions to cause one or more prompts to be communicated (604) to the user to aid in modifying the first emotion score; optionally wherein the one or more prompts include a visual prompt, an audio prompt, or both.

3. The system of claim 2, wherein the visual prompt includes light emitted from a light source (180), optionally wherein the light has a color, an intensity, and a pattern of emission, further optionally wherein the visual prompt includes modifying the color of the light, modifying the intensity of the light, modifying the pattern of emission of the light, or any combination thereof.

4. The system of claim 2 or claim 3, wherein the one or more prompts include a breathing exercise to aid in modifying a respiration rate of the user, optionally wherein the determining the first emotion score associated with the user includes determining a respiration rate associated with the user based at least in part on the first physiological data, further optionally wherein one or more light pulses are emitted from the light source at a predetermined frequency to aid in modifying the respiration rate associated with the user.

5. The system of claim 3 or claim 4, wherein the light source (180) is physically coupled to or integrated in a user device (170), optionally wherein the light source (180) is a display device (172) of the user device (170); or
the light source (180) physically coupled to or integrated in a portion of the respiratory therapy system (120), optionally wherein the portion of the respiratory therapy system (120) is a user interface (124), a conduit (126), or a respiratory therapy device (122).

6. The system of any one of claims 2 to 5, wherein the audio prompt is communicated to the user via a transducer (142), optionally wherein the transducer (142) is physically coupled to or integrated in the respiratory therapy system (120).

7. The system of any one of claims 1 to 6, wherein the determining the first emotion score associated with the user includes determining a movement, a respiration rate, a respiration rate variability, a respiration depth, a tidal volume, an inspiration amplitude, an inspiration duration, an expiration amplitude, an expiration duration, an inspiration-expiration ratio, a heart rate, a heart rate variability, a cardiac waveform, perspiration, blood oxygenation, blood pressure, peripheral arterial tone, cardiogenic oscillations, a galvanic skin response, a sympathetic nervous system response, a skin temperature, an ambient temperature, photoplethysmography, pulse transit time, a core body temperature, a trend associated with the respiration rate, a trend associated with the heart rate, a trend associated with the galvanic skin response, or any combination thereof.

8. The system of any one of claims 1 to 7, wherein the modification of the one or more settings of the respiratory therapy system(120) causes a respiratory therapy device (122) of the respiratory therapy system (120) to supply pressurized air at a predetermined pressure; and/or
wherein the first physiological data is received when the user is wearing a user interface (124) of the respiratory therapy system (120), optionally wherein the respiratory therapy system (120) supplies pressurized air at a first predetermined pressure prior to determining that the first emotion score satisfies the predetermined condition, further optionally wherein the modification of the one or more settings of the respiratory therapy system (120) causes the respiratory therapy system (120) to supply pressurized air a second predetermined pressure that is different than the first predetermined pressure, yet further optionally wherein the second predetermined pressure is greater than the first predetermined pressure; and/or
wherein the determining that the first emotion score satisfies the predetermined condition includes determining that the first emotion score is less than the predetermined threshold.

9. The system of any one of claims 1 to 8, wherein the first physiological data is generated by one or more sensors (130), optionally wherein at least one of the one or more sensors (130) is physically coupled to or integrated in the respiratory therapy system (120), and/or is physically coupled to or integrated in a user device (170) or an activity tracker (190) that is separate and distinct from the respiratory therapy system (120); and/or
wherein the respiratory therapy system (120) is a continuous positive airway pressure (CPAP) system; and/or
wherein at least a portion of the first physiological data is associated with at least a portion of a first sleep session of the user.

10. The system of any one of claims 1 to 9, wherein the control system (110) is further configured to execute machine-readable instructions to:
receive second physiological data associated with the user subsequent to the implementation of the determined modification of one or more settings of the respiratory therapy system (120), the second physiological data being associated with a first sleep session of the user; and
determine a second emotion score associated with the user based at least in part on the second physiological data;
optionally
cause an indication of the first emotion score, the second emotion score, or both to be communicated to the user, a third party, or both, during or subsequent to the first sleep session; and/or
cause further modification of the one or more settings of the respiratory therapy system (120) responsive to determining that the second emotion score does not satisfy the predetermined condition; and/or
modify one or more settings of one or more Internet of Things (IoT) devices based at least in part on the determined second emotion score.

11. The system of any one of claims 1 to 10, wherein the control system (110) is further configured to execute machine-readable instructions to determine a therapy recommendation for the user based at least in part on the first emotion score and cause an indication of the recommendation to be communicated to the user, a third party, or both,
optionally wherein the therapy recommendation includes a recommendation to modify a type of user interface (124) for the respiratory therapy system (120), further optionally wherein the type of user interface (124) for the respiratory therapy system (120) is a full face mask, a nasal pillows mask, or a nasal mask; and/or
optionally wherein the therapy recommendation includes a medication recommendation, or
optionally wherein the therapy recommendation includes a recommendation to use an alternative medical device, optionally wherein the alternative medical device includes a mandibular repositioning device, a neurostimulatory device, or both, further optionally wherein the alternative medical device includes a mandibular repositioning device, a neurostimulatory device, or both.

12. The system of any one of claims 1 to 11, wherein the first physiological data is generated during at least a portion of a first sleep session and the user uses the respiratory therapy system (120) with the modified one or more settings during at least a portion of the first sleep session,
optionally wherein the control system (110) is further configured to execute machine-readable instructions to:
receive second physiological data associated with the user during at least a portion of a second sleep session that is subsequent to the first sleep session;
determine a second emotion score associated with the user based at least in part on the second physiological data, the second emotion score being determined relative to the first emotion score; and
cause one or more prompts to be communicated to the user to aid in modifying the determined second emotion score
further optionally wherein the one or more prompts for aiding in modifying the determined second emotion score are different than the one or more prompts for aiding in modifying the determined first emotion score.

13. The system of claim 12, wherein the control system (110) is further configured to execute machine-readable instructions to cause a modification of one or more settings of the respiratory therapy system (120) for the second sleep session based at least in part on the first emotion score, the second emotion score, or both; or
wherein the control system (110) is further configured to execute machine-readable instructions to:
determine one or more first sleep-related parameters associated with the first sleep session based at least in part on the first physiological data;
determine one or more second sleep-related parameters associated with the second sleep session based at least in part on the second physiological data; and
cause an indication of at least one of the one or more first sleep-related parameters, at least one of the one or more second sleep-related parameters, or both to be communicated to the user, a third party, or both subsequent to the second sleep session.

14. The system of any one of claims 1 to 13, wherein the respiratory therapy system (120) includes a user interface (124) configured to engage a portion the user, the user interface (124) including a vent, optionally wherein the implementation of the determined modification of the one or more settings of the respiratory therapy system (120) includes modifying a position of the vent, further optionally wherein the modifying the position of the vent includes causing the vent to move from a generally open position towards a generally closed position; and/or
wherein the one or more prompts include one or more prompts to assemble a user interface of the respiratory therapy system.

15. A computer program product comprising instructions which, when executed by a computer, cause the computer to carry out the method of:
receiving (601) first physiological data associated with a user;
determining (602) a first emotion score associated with the user based at least in part on the first physiological data, the first emotion score being determined relative to a prior emotion score at which the user was able to fall asleep; and
responsive to determining that the first emotion score satisfies a predetermined condition, determining (605) a modification of one or more settings of the respiratory therapy system (120).

## Patentansprüche

1. System, umfassend:
ein Atemtherapie-System (120);
einen Arbeitsspeicher (114), der maschinenlesbare Anweisungen darauf gespeichert aufweist:
ein Steuersystem (110), das einen oder mehrere Prozessoren (112) beinhaltet, die dazu ausgelegt sind, die maschinenlesbaren Anweisungen auszuführen, um:
erste physiologische Daten zu empfangen (601), die einem Benutzer zugeordnet sind;
einen ersten Gefühlswert, der dem Benutzer zugeordnet ist, zumindest teilweise auf der Grundlage der ersten physiologischen Daten zu bestimmen (602), wobei der erste Gefühlswert relativ zu einem früheren Gefühlswert bestimmt wird, bei dem der Benutzer in der Lage war, einzuschlafen; und
in Ansprechen auf das Bestimmen, dass der erste Gefühlswert eine vorbestimmte Bedingung erfüllt, eine Modifikation einer oder mehrerer Einstellungen des Atemtherapie-Systems (120) zu bestimmen (605).

2. System nach Anspruch 1, wobei das Steuersystem (110) ferner dazu ausgestaltet ist, maschinenlesbare Anweisungen auszuführen, um zu veranlassen, dass eine oder mehrere Aufforderungen an den Benutzer kommuniziert (604) werden, bei der Modifikation des ersten Gefühlswerts zu helfen; wobei optional die eine oder die mehreren Aufforderungen eine visuelle Aufforderung, eine akustische Aufforderung oder beides beinhalten.

3. System nach Anspruch 2, wobei die visuelle Aufforderung von einer Lichtquelle (180) emittiertes Licht beinhaltet, wobei das Licht optional eine Farbe, eine Intensität, und ein Emissionsmuster aufweist, wobei die visuelle Aufforderung ferner optional das Modifizieren der Farbe des Lichts, das Modifizieren der Intensität des Lichts, das Modifizieren des Emissionsmusters des Lichts oder eine beliebige Kombination davon beinhaltet.

4. System nach Anspruch 2 oder Anspruch 3, wobei die eine oder die mehreren Aufforderungen eine Atemübung beinhalten, um dabei zu helfen, eine Atmungsrate des Benutzers zu modifizieren, wobei das Bestimmen des ersten Gefühlswerts, der dem Benutzer zugeordnet ist, optional das Bestimmen einer Atmungsrate, die dem Benutzer zugeordnet ist, zumindest zum Teil auf Grundlage der ersten physiologischen Daten beinhaltet, wobei ferner optional ein oder mehrere Lichtimpulse mit einer vorbestimmten Frequenz von der Lichtquelle emittiert werden, um dabei zu helfen, die Atmungsrate, die dem Benutzer zugeordnet ist, zu modifizieren.

5. System nach Anspruch 3 oder Anspruch 4, wobei die Lichtquelle (180) physisch mit einer Benutzervorrichtung (170) gekoppelt oder in diese integriert ist, wobei optional die Lichtquelle (180) eine Anzeigevorrichtung (172) der Benutzervorrichtung (170) ist; oder
die Lichtquelle (180) physisch mit einem Abschnitt des Atemtherapie-Systems (120) gekoppelt oder in diesen integriert ist, wobei optional der Abschnitt des Atemtherapie-Systems (120) eine Benutzerschnittstelle (124), eine Leitung (126), oder eine Atemtherapie-Vorrichtung (122) ist.

6. System nach einem der Ansprüche 2 bis 5, wobei die akustische Aufforderung an den Benutzer über einen Wandler (142) kommuniziert wird, wobei der Wandler (142) optional physisch mit dem Atemtherapie-System (120) gekoppelt oder in dieses integriert ist.

7. System nach einem der Ansprüche 1 bis 6, wobei das Bestimmen des ersten Gefühlswerts, der dem Benutzer zugeordnet ist, das Bestimmen einer Bewegung, einer Atmungsrate, einer Atmungsratenvariabilität, einer Atmungstiefe, eines Tidalvolumens, einer Inspirationsamplitude, einer Inspirationsdauer, einer Exspirationsamplitude, einer Exspirationsdauer, eines Inspirations-Exspirations-Verhältnisses, einer Herzrate, einer Herzratenvariabilität, einer kardialen Wellenform, der Transpiration, der Blutsauerstoffversorgung, des Blutdrucks, des peripheren arteriellen Tonus, der kardiogenen Oszillationen, einer galvanischen Hautreaktion, einer Reaktion des sympathischen Nervensystems, einer Hauttemperatur, einer Umgebungstemperatur, der Photoplethysmographie, der Pulsdurchlaufzeit, einer Körperkerntemperatur, eines Trends, der der Atmungsrate zugeordnet ist, eines Trends, der der Herzrate zugeordnet ist, eines Trends, der der galvanischen Hautreaktion zugeordnet ist, oder einer beliebigen Kombination davon beinhaltet.

8. System nach einem der Ansprüche 1 bis 7, wobei die Modifikation der einen oder der mehreren Einstellungen des Atemtherapie-Systems (120) eine Atemtherapie-Vorrichtung (122) des Atemtherapie-Systems (120) veranlasst, druckbeaufschlagte Luft mit einem vorbestimmten Druck zuzuführen; und/oder
wobei die ersten physiologischen Daten empfangen werden, wenn der Benutzer eine Benutzerschnittstelle (124) des Atemtherapie-Systems (120) trägt, wobei das Atemtherapie-System (120) optional vor dem Bestimmen, dass der erste Gefühlswert die vorbestimmte Bedingung erfüllt, druckbeaufschlagte Luft mit einem vorbestimmten Druck zuführt, wobei ferner die Modifikation der einen oder der mehreren Einstellungen des Atemtherapie-Systems (120) optional das Atemtherapie-System (120) veranlasst, druckbeaufschlagte Luft mit einem zweiten vorbestimmten Druck, der sich von dem ersten vorbestimmten Druck unterscheidet, zuzuführen, wobei ferner der zweite vorbestimmte Druck optional höher ist als der erste vorbestimmte Druck; und/oder
wobei das Bestimmen, dass der erste Gefühlswert die vorbestimmte Bedingung erfüllt, das Bestimmen beinhaltet, dass der erste Gefühlswert niedriger ist als die vorbestimmte Schwelle.

9. System nach einem der Ansprüche 1 bis 8, wobei die ersten physiologischen Daten durch einen oder mehrere Sensoren (130) erzeugt werden, wobei optional zumindest einer der einen oder mehreren Sensoren (130) physisch mit dem Atemtherapie-System (120) gekoppelt oder in dieses integriert ist, und/oder physisch mit einer BenutzerVorrichtung (170) oder einem Aktivitätstracker (190), die/der sich von dem Atemtherapie-System (120) unterscheidet, gekoppelt oder in diese/n integriert ist; und/oder
wobei das Atemtherapie-System (120) ein System mit kontinuierlichem positivem Atemwegsdruck (CPAP) ist; und/oder
wobei zumindest ein Teil der ersten physiologischen Daten zumindest einem Abschnitt einer ersten Schlafsitzung des Benutzers zugeordnet ist.

10. System nach einem der Ansprüche 1 bis 9, wobei das Steuersystem (110) ferner dazu ausgestaltet ist, maschinenlesbare Anweisungen auszuführen, um:
auf die Implementierung der bestimmten Modifikation einer oder mehrerer Einstellungen des Atemtherapie-Systems (120) folgend zweite physiologische Daten zu empfangen, die dem Benutzer zugeordnet sind, wobei die zweiten physiologischen Daten einer ersten Schlafsitzung des Benutzers zugeordnet sind; und
einen zweiten Gefühlswert, der dem Benutzer zugeordnet ist, zumindest teilweise auf der Grundlage der zweiten physiologischen Daten zu bestimmen; optional
während oder nach der ersten Schlafsitzung eine Angabe des ersten Gefühlswerts, des zweiten Gefühlswerts oder beides an den Benutzer, eine dritte Partei oder beide zu kommunizieren; und/oder
in Ansprechen auf das Bestimmen, dass der zweite Gefühlswert die vorbestimmte Bedingung nicht erfüllt, eine weitere Modifikation der einen oder der mehreren Einstellungen des Atemtherapie-Systems (120) zu veranlassen; und/oder
eine oder mehrere Einstellungen einer oder mehrerer Internet-of-Things- bzw. loT-Vorrichtungen zumindest teilweise auf Grundlage des bestimmten zweiten Gefühlswerts zu modifizieren.

11. System nach einem der Ansprüche 1 bis 10, wobei das Steuersystem (110) ferner dazu ausgestaltet ist, maschinenlesbare Anweisungen auszuführen, um eine Therapieempfehlung für den Benutzer zumindest teilweise auf Grundlage des ersten Gefühlswerts zu bestimmen, und zu veranlassen, dass eine Angabe der Empfehlung an den Benutzer, eine dritte Partei oder beide kommuniziert wird,
wobei die Therapieempfehlung optional eine Empfehlung beinhaltet, einen Typ von Benutzerschnittstelle (124) für das Atemtherapie-System (120) zu modifizieren, wobei der Typ der Benutzerschnittstelle (124) für das Atemtherapie-System (120) optional ferner eine Vollgesichtsmaske, eine Nasenpolstermaske oder eine Nasenmaske ist; und/oder
wobei die Therapieempfehlung optional eine Medikationsempfehlung beinhaltet, oder
wobei die Therapieempfehlung optional eine Empfehlung beinhaltet, eine alternative medizinische Vorrichtung zu verwenden, wobei die alternative medizinische Vorrichtung eine Vorrichtung zur Repositionierung des Unterkiefers, eine neurostimulatorische Vorrichtung oder beides beinhaltet, wobei die alternative medizinische Vorrichtung ferner optional eine Vorrichtung zur Repositionierung des Unterkiefers, eine neurostimulatorische Vorrichtung oder beides beinhaltet.

12. System nach einem der Ansprüche 1 bis 11, wobei die ersten physiologischen Daten während zumindest eines Abschnitts einer ersten Schlafsitzung erzeugt werden und der Benutzer das Atemtherapie-System (120) mit den einen oder mehreren modifizierten Einstellungen während zumindest eines Abschnitts der ersten Schlafsitzung verwendet,
wobei optional das Steuersystem (110) ferner dazu ausgestaltet ist, maschinenlesbare Anweisungen auszuführen, um:
zweite physiologische Daten, die dem Benutzer zugeordnet sind, während zumindest eines Abschnitts einer zweiten Schlafsitzung, die auf die erste Schlafsitzung folgt, zu empfangen;
einen zweiten Gefühlswert, der dem Benutzer zugeordnet ist, zumindest teilweise auf der Grundlage der zweiten physiologischen Daten zu bestimmen, wobei der zweite Gefühlswert relativ zu dem ersten Gefühlswert bestimmt wird; und
zu veranlassen, dass eine oder mehrere Aufforderungen an den Benutzer kommuniziert werden, dabei zu helfen, den bestimmten zweiten Gefühlswert zu modifizieren, wobei ferner die eine oder die mehreren Aufforderungen, dabei zu helfen, den bestimmten zweiten Gefühlswert zu modifizieren, sich optional von der einen oder den mehreren Aufforderungen, dabei zu helfen, den bestimmten ersten Gefühlswert zu modifizieren, unterscheiden.

13. System nach Anspruch 12, wobei das Steuersystem (110) ferner dazu ausgestaltet ist, maschinenlesbare Anweisungen auszuführen, um eine Modifikation von einer oder mehreren Einstellungen des Atemtherapie-Systems (120) für die zweite Schlafsitzung zumindest teilweise auf Grundlage des ersten Gefühlswerts, des zweiten Gefühlswerts oder beider zu veranlassen; oder
wobei das Steuersystem (110) ferner dazu ausgestaltet ist, maschinenlesbare Anweisungen auszuführen, um:
einen oder mehrere erste schlafbezogene Parameter, die der ersten Schlafsitzung zugeordnet sind, zumindest teilweise auf Grundlage der ersten physiologischen Daten zu bestimmen;
einen oder mehrere zweite schlafbezogene Parameter, die der zweiten Schlafsitzung zugeordnet sind, zumindest teilweise auf Grundlage der zweiten physiologischen Daten zu bestimmen; und
zu veranlassen, dass auf die zweite Schlafsitzung folgend eine Angabe zumindest eines von dem einem oder den mehreren ersten schlafbezogenen Parametern, zumindest eines von dem einem oder den mehreren zweiten schlafbezogenen Parametern, oder beider an den Benutzer, eine dritte Partei oder beide kommuniziert wird.

14. System nach einem der Ansprüche 1 bis 13, wobei das Atemtherapie-System (120) eine Benutzerschnittstelle (124) beinhaltet, die dazu ausgestaltet ist, mit einem Teil des Benutzers in Eingriff zu stehen, wobei die Benutzerschnittstelle (124) eine Entlüftungsöffnung beinhaltet, wobei optional die Implementierung der bestimmten Modifikation der einen oder der mehreren Einstellungen des Atemtherapie-Systems (120) das Modifizieren einer Stellung der Entlüftungsöffnung beinhaltet, wobei ferner das Modifizieren der Stellung der Entlüftungsöffnung beinhaltet, die Entlüftungsöffnung zu veranlassen, sich von einer allgemein offenen Stellung zu einer allgemein geschlossenen Stellung hin zu bewegen; und/oder
wobei die eine oder die mehreren Aufforderungen eine oder mehrere Aufforderungen beinhalten, eine Benutzerschnittstelle des Atemtherapie-Systems anzulegen.

15. Computerprogrammprodukt, umfassend Anweisungen, die, wenn sie von einem Computer ausgeführt werden, den Computer veranlassen, folgendes Verfahren durchzuführen:
Empfangen (601) erster physiologischer Daten, die einem Benutzer zugeordnet sind;
Bestimmen (602) eines ersten Gefühlswerts, der dem Benutzer zugeordnet ist, zumindest teilweise auf der Grundlage der ersten physiologischen Daten, wobei der erste Gefühlswert relativ zu einem früheren Gefühlswert bestimmt wird, bei dem der Benutzer in der Lage war, einzuschlafen; und
Bestimmen (605) einer Modifikation einer oder mehrerer Einstellungen des Atemtherapie-Systems (120) in Ansprechen auf das Bestimmen, dass der erste Gefühlswert eine vorbestimmte Bedingung erfüllt.

## Revendications

1. Système comprenant :
un système de thérapie respiratoire (120) ;
une mémoire (114) dans laquelle sont stockées des instructions lisibles par machine ; et
un système de commande (110) incluant un ou plusieurs processeurs (112) configurés pour exécuter les instructions lisibles par machine pour :
recevoir (601) des premières données physiologiques associées à un utilisateur ;
déterminer (602) un premier score d'émotion associé à l'utilisateur sur la base au moins en partie des premières données physiologiques, le premier score d'émotion étant déterminé par rapport à un score d'émotion antérieur auquel l'utilisateur a pu s'endormir ; et
en réponse à la détermination que le premier score d'émotion satisfait une condition prédéterminée, déterminer (605) une modification d'un ou de plusieurs réglages du système de thérapie respiratoire (120).

2. Système selon la revendication 1, dans lequel le système de commande (110) est en outre configuré pour exécuter des instructions lisibles par machine afin de provoquer la communication (604) d'une ou de plusieurs invites à l'utilisateur pour aider à modifier le premier score d'émotion ; éventuellement, dans lequel la ou les plusieurs invites incluent une invite visuelle, une invite audio ou les deux.

3. Système selon la revendication 2, dans lequel l'invite visuelle inclut une lumière émise par une source lumineuse (180), éventuellement dans lequel la lumière a une couleur, une intensité et un motif d'émission, en outre éventuellement dans lequel l'invite visuelle inclut la modification de la couleur de la lumière, la modification de l'intensité de la lumière, la modification du motif d'émission de la lumière, ou toute combinaison de celles-ci.

4. Système selon la revendication 2 ou la revendication 3, dans lequel la ou les plusieurs invites incluent un exercice de respiration pour aider à modifier un rythme respiratoire de l'utilisateur, éventuellement dans lequel la détermination du premier score d'émotion associé à l'utilisateur inclut la détermination d'un rythme respiratoire associé à l'utilisateur sur la base au moins en partie des premières données physiologiques, en outre éventuellement dans lequel une ou plusieurs impulsions lumineuses sont émises par la source lumineuse à une fréquence prédéterminée pour aider à modifier le rythme respiratoire associé à l'utilisateur.

5. Système selon la revendication 3 ou 4, dans lequel la source lumineuse (180) est physiquement couplée ou intégrée à un dispositif utilisateur (170), éventuellement dans lequel la source lumineuse (180) est un dispositif d'affichage (172) du dispositif utilisateur (170) ; ou
la source lumineuse (180) physiquement couplée ou intégrée à une partie du système de thérapie respiratoire (120), éventuellement dans lequel la partie du système de thérapie respiratoire (120) est une interface utilisateur (124), un conduit (126) ou un dispositif de thérapie respiratoire (122).

6. Système selon l'une quelconque des revendications 2 à 5, dans lequel l'invite audio est communiquée à l'utilisateur via un transducteur (142), éventuellement dans lequel le transducteur (142) est physiquement couplé ou intégré au système de thérapie respiratoire (120).

7. Système selon l'une quelconque des revendications 1 à 6, dans lequel la détermination du premier score d'émotion associé à l'utilisateur inclut la détermination d'un mouvement, d'un rythme respiratoire, d'une variabilité du rythme respiratoire, d'une profondeur de respiration, d'un volume courant, d'une amplitude d'inspiration, d'une durée d'inspiration, d'une amplitude d'expiration, d'une durée d'expiration, d'un rapport inspiration-expiration, d'une fréquence cardiaque, d'une variabilité de fréquence cardiaque, d'une forme d'onde cardiaque, de la transpiration, de l'oxygénation du sang, de la pression artérielle, du tonus artériel périphérique, des oscillations cardiogéniques, d'une réponse galvanique de la peau, d'une réponse du système nerveux sympathique, d'une température de peau, d'une température ambiante, d'une photopléthysmographie, d'un temps de transit d'impulsion, d'une température corporelle centrale, d'une tendance associée au rythme respiratoire, d'une tendance associée à la fréquence cardiaque, d'une tendance associée à la réponse galvanique de la peau, ou de toute combinaison de celles-ci.

8. Système selon l'une quelconque des revendications 1 à 7, dans lequel la modification d'un ou de plusieurs réglages du système de thérapie respiratoire (120) amène un dispositif de thérapie respiratoire (122) du système de thérapie respiratoire (120) à fournir de l'air sous pression à une pression prédéterminée ; et/ou
dans lequel les premières données physiologiques sont reçues lorsque l'utilisateur porte une interface utilisateur (124) du système de thérapie respiratoire (120), éventuellement dans lequel le système de thérapie respiratoire (120) fournit de l'air sous pression à une première pression prédéterminée avant de déterminer que le premier score d'émotion satisfait la condition prédéterminée, en outre éventuellement dans lequel la modification du ou des plusieurs réglages du système de thérapie respiratoire (120) amène le système de thérapie respiratoire (120) à fournir de l'air sous pression à une seconde pression prédéterminée qui est différente de la première pression prédéterminée, encore en outre éventuellement dans lequel la seconde pression prédéterminée est supérieure à la première pression prédéterminée ; et/ou
dans lequel la détermination que le premier score d'émotion satisfait la condition prédéterminée inclut la détermination que le premier score d'émotion est inférieur au seuil prédéterminé.

9. Système selon l'une quelconque des revendications 1 à 8, dans lequel les premières données physiologiques sont générées par un ou plusieurs capteurs (130), éventuellement dans lequel au moins un du ou des plusieurs capteurs (130) est physiquement couplé ou intégré au système de thérapie respiratoire (120), et/ou est physiquement couplé ou intégré à un dispositif utilisateur (170) ou à un dispositif de suivi d'activité (190) qui est séparé et distinct du système de thérapie respiratoire (120) ; et/ou
dans lequel le système de thérapie respiratoire (120) est un système de ventilation en pression positive continue (CPAP) ; et/ou
dans lequel au moins une partie des premières données physiologiques est associée à au moins une partie d'une première session de sommeil de l'utilisateur.

10. Système selon l'une quelconque des revendications 1 à 9, dans lequel le système de commande (110) est en outre configuré pour exécuter des instructions lisibles par machine pour :
recevoir des secondes données physiologiques associées à l'utilisateur suite à la mise en œuvre de la modification déterminée d'un ou de plusieurs réglages du système de thérapie respiratoire (120), les secondes données physiologiques étant associéesà une première session de sommeil de l'utilisateur ; et
déterminer un second score d'émotion associé à l'utilisateur sur la base au moins en partie des secondes données physiologiques ; éventuellement
provoquer la communication d'une indication du premier score d'émotion, du second score d'émotion, ou des deux, à l'utilisateur, à un tiers, ou aux deux, pendant ou après la première session de sommeil ; et/ou
provoquer une modification supplémentaire d'un ou de plusieurs réglages du système de thérapie respiratoire (120) en réponse à la détermination que le second score d'émotion ne satisfait pas la condition prédéterminée ; et/ou
modifier un ou plusieurs réglages d'un ou de plusieurs dispositifs de l'Internet des objets (loT) sur la base au moins en partie du second score d'émotion déterminé.

11. Système selon l'une quelconque des revendications 1 à 10, dans lequel le système de commande (110) est en outre configuré pour exécuter des instructions lisibles par machine afin de déterminer une recommandation thérapeutique pour l'utilisateur sur la base au moins en partie du premier score d'émotion, et pour provoquer la communication d'une indication de la recommandation à l'utilisateur, à un tiers, ou aux deux.
éventuellement dans lequel la recommandation thérapeutique inclut une recommandation de modification d'un type d'interface utilisateur (124) pour le système de thérapie respiratoire (120), en outre éventuellement dans lequel le type d'interface utilisateur (124) pour le système de thérapie respiratoire (120) est un masque facial complet, un masque narinaire ou un masque nasal ; et/ou
éventuellement dans lequel la recommandation thérapeutique inclut une recommandation de médicament, ou
éventuellement dans lequel la recommandation thérapeutique inclut une recommandation d'utiliser un dispositif médical alternatif, éventuellement dans lequel le dispositif médical alternatif inclut un dispositif de repositionnement mandibulaire, un dispositif neurostimulateur, ou les deux, en outre éventuellement dans lequel le dispositif médical alternatif inclut un dispositif de repositionnement mandibulaire, un dispositif neurostimulateur, ou les deux.

12. Système selon l'une quelconque des revendications 1 à 11, dans lequel les premières données physiologiques sont générées pendant au moins une partie d'une première session de sommeil et l'utilisateur utilise le système de thérapie respiratoire (120) avec le ou les plusieurs réglages modifiés pendant au moins une partie de la première session de sommeil,
éventuellement dans lequel le système de commande (110) est en outre configuré pour exécuter des instructions lisibles par machine pour :
recevoir des secondes données physiologiques associées à l'utilisateur pendant au moins une partie d'une seconde session de sommeil qui est postérieure à la première session de sommeil ;
déterminer un second score d'émotion associé à l'utilisateur sur la base au moins en partie des secondes données physiologiques, le second score d'émotion étant déterminé par rapport au premier score d'émotion ; et
provoquer la communication d'une ou de plusieurs invites à l'utilisateur pour aider à modifier le second score d'émotion déterminé, en outre éventuellement dans lequel la ou les plusieurs invites pour aider à modifier le second score d'émotion déterminé sont différentes de la ou des plusieurs invites pour aider à modifier le premier score d'émotion déterminé.

13. Système selon la revendication 12, dans lequel le système de commande (110) est en outre configuré pour exécuter des instructions lisibles par machine afin de provoquer la modification d'un ou de plusieurs réglages du système de thérapie respiratoire (120) pour la seconde session de sommeil sur la base au moins en partie du premier score d'émotion, du second score d'émotion, ou des deux ; ou
dans lequel le système de commande (110) est en outre configuré pour exécuter des instructions lisibles par machine pour :
déterminer un ou plusieurs premiers paramètres liés au sommeil associés à la première session de sommeil sur la base au moins en partie des premières données physiologiques ;
déterminer un ou plusieurs seconds paramètres liés au sommeil associés à la seconde session de sommeil sur la base au moins en partie des secondes données physiologiques ; et
provoquer la communication d'une indication d'au moins un du ou des plusieurs premiers paramètres liés au sommeil, d'au moins un du ou des plusieurs seconds paramètres liés au sommeil, ou des deux, à l'utilisateur, à un tiers, ou aux deux après la seconde session de sommeil.

14. Système selon l'une quelconque des revendications 1 à 13, dans lequel le système de thérapie respiratoire (120) inclut une interface utilisateur (124) configurée pour s'engager avec une partie de l'utilisateur, l'interface utilisateur (124) incluant un évent, éventuellement dans lequel la mise en œuvre de la modification déterminée du ou des plusieurs réglages du système de thérapie respiratoire (120) inclut la modification de la position de l'évent, en outre éventuellement dans lequel la modification de la position de l'évent inclut l'entraînement du déplacement de l'évent d'une position généralement ouverte vers une position généralement fermée ; et/ou
dans lequel la ou les plusieurs invites incluent une ou plusieurs invites pour assembler une interface utilisateur du système de thérapie respiratoire.

15. Produit de programme informatique comprenant des instructions qui, lorsqu'elles sont exécutées par un ordinateur, amènent l'ordinateur à réaliser le procédé de :
réception (601) de premières données physiologiques associées à un utilisateur ;
détermination (602) d'un premier score d'émotion associé à l'utilisateur sur la base au moins en partie des premières données physiologiques, le premier score d'émotion étant déterminé par rapport à un score d'émotion antérieur auquel l'utilisateur a pu s'endormir ; et
en réponse à la détermination que le premier score d'émotion satisfait une condition prédéterminée, détermination (605) d'une modification d'un ou de plusieurs réglages du système de thérapie respiratoire (120).
